# EUROPEAN PATENT SPECIFICATION

(11) **EP 4 130 273 B1**
(45) Date of publication and mention of the grant of the patent: **12.06.2024**
(21) Application number: 22181704.2
(22) Date of filing: 28.12.2010
(51) Int. Cl.: C12N 15/44, A61P 31/12, A61P 31/16, A61P 37/04, C12N 1/12, C12P 21/02

(54) **PRODUCTION OF HETEROLOGOUS POLYPEPTIDES IN MICROALGAE, MICROALGAL EXTRACELLULAR BODIES, COMPOSITIONS, AND METHODS OF MAKING AND USES THEREOF**
HERSTELLUNG HETEROLOGER POLYPEPTIDE IN MIKROALGEN, EXTRAZELLULÄRE ALGENKÖRPER, ZUSAMMENSETZUNGEN UND VERFAHREN ZUR HERSTELLUNG SOWIE VERWENDUNGEN DAVON
PRODUCTION DE POLYPEPTIDES HÉTÉROLOGUES DANS DES MICRO-ALGUES, CORPS EXTRACELLULAIRE DE MICRO-ALGUES, COMPOSITIONS ET PROCÉDÉS DE FABRICATION ET LEURS UTILISATIONS

(30) Priority: 28.12.2009 US 29046909 P; 28.12.2009 US 29044109 P; 12.11.2010 US 41335310 P
(43) Date of publication of application: 08.02.2023
(62) Divisional of application: 18205786.9
(73) Proprietor: Sanofi Vaccine Technologies, S.A.S., 93230 Paris (FR)
(72) Inventor: BAYNE, Anne-Cecile V., Ellicott City, MD 21043 (US); LIPPMEIER, James Casey, Columbia, MD 21045 (US); APT, Kirk Emil, deceased (US); ZIRKLE, Ross Eric, Mt. Airy, MD 21771 (US)
(74) Representative: Mathys & Squire

(56) References cited:
- EP-A1- 2 090 648
- WO-A1-95/18861
- WO-A2-02/083869
- WO-A2-2006/013572
- WO-A2-2009/113795
- AU-A1- 2007 249 124
- US-A1- 2006 275 904
- US-A1- 2006 286 650
- US-A1- 2008 022 422
- YONGKIETTRAKUL S ET AL: "Avian influenza A/H5N1 neuraminidase expressed in yeast with a functional head domain", JOURNAL OF VIROLOGICAL METHODS, ELSEVIER BV, NL, vol. 156, no. 1-2, 1 March 2009 (2009-03-01), pages 44-51, XP025941308, ISSN: 0166-0934, DOI: 10.1016/J.JVIROMET.2008.10.025 [retrieved on 2008-12-11]
- SIRIPORNADULSIL SURASAK ET AL: "Microalgal vaccines", ADVANCES IN EXPERIMENTAL MEDICINE AND BIOLOGY,, 1 January 2007 (2007-01-01), pages 122-128, XP009097595, ISSN: 0065-2598, DOI: 10.1007/978-0-387-75532-8_11
- MAYFIELD ET AL: "Chlamydomonas reinhardtii chloroplasts as protein factories", CURRENT OPINION IN BIOTECHNOLOGY, LONDON, GB, vol. 18, no. 2, 17 April 2007 (2007-04-17) , pages 126-133, XP022032091, ISSN: 0958-1669, DOI: 10.1016/J.COPBIO.2007.02.001
- WATANABE SHINJI ET AL: "Influenza B Virus BM2 Protein Is Transported through the trans- Golgi Network as an Integral Membrane Protein", JOURNAL OF VIROLOGY, vol. 77, no. 19, 1 October 2003 (2003-10-01), pages 10630-10637, XP093007585, US ISSN: 0022-538X, DOI: 10.1128/JVI.77.19.10630-10637.2003 Retrieved from the Internet: URL:https://www.ncbi.nlm.nih.gov/pmc/artic les/PMC228492/pdf/0633.pdf>
- ANNE-CÉCILE V. BAYNE ET AL: "Vaccination against Influenza with Recombinant Hemagglutinin Expressed by Schizochytrium sp. Confers Protective Immunity", PLOS ONE, vol. 8, no. 4, 1 January 2013 (2013-01-01) , pages e61790-e61790, XP055062273, ISSN: 1932-6203, DOI: 10.1371/journal.pone.0061790

## Description

### BACKGROUND OF THE INVENTION

### Field of the Present Disclosure

The present disclosure relates to recombinant microalgal cells and their use in heterologous polypeptide production, methods of production of heterologous polypeptides in microalgal extracellular bodies, microalgal extracellular bodies comprising heterologous polypeptides, and compositions comprising the same.

### Background Art

Advancements in biotechnology and molecular biology have enabled the production of proteins in microbial, plant, and animal cells, many of which were previously available only by extraction from tissues, blood, or urine of humans and other animals. Biologies that are commercially available today are typically manufactured either in mammalian cells, such as Chinese Hamster Ovary (CHO) cells, or in microbial cells, such as yeast or *E. coli* cell lines.

Production of proteins via the fermentation of microorganisms presents several advantages over existing systems such as plant and animal cell culture. For example, microbial fermentation-based processes can offer: (i) rapid production of high concentration of protein; (ii) the ability to use sterile, well-controlled production conditions (such as Good Manufacturing Practice (GMP) conditions); (iii) the ability to use simple, chemically defined growth media allowing for simpler fermentations and fewer impurities; (iv) the absence of contaminating human or animal pathogens; and (v) the ease of recovering the protein (e.g., via isolation from the fermentation media). In addition, fermentation facilities are typically less costly to construct than cell culture facilities.

Microalgae, such as thraustochytrids of the phylum Labyrinthulomycota, can be grown with standard fermentation equipment, with very short culture cycles (e.g., 1-5 days), inexpensive defined media and minimal purification, if any. Furthermore, certain microalgae, e.g., *Schizochytrium,* have a demonstrated history of safety for food applications of both the biomass and lipids derived therefrom. For example, DHA-enriched triglyceride oil from this microorganism has received GRAS (Generally Recognized as Safe) status from the U.S. Food and Drug Administration.

Microalgae have been shown to be capable of expressing recombinant proteins. For example, U.S. Patent No. 7,001,772 disclosed the first recombinant constructs suitable for transforming thraustochytrids, including members of the genus *Schizochytrium.* This publication disclosed, among other things, *Schizochytrium* nucleic acid and amino acid sequences for an acetolactate synthase, an acetolactate synthase promoter and terminator region, an α-tubulin promoter, a promoter from a polyketide synthase (PKS) system, and a fatty acid desaturase promoter. U.S. Publ. Nos. 2006/0275904 and 2006/0286650 subsequently disclosed *Schizochytrium* sequences for actin, elongation factor 1 alpha (ef1α), and glyceraldehyde 3-phosphate dehydrogenase (gapdh) promoters and terminators.

A continuing need exists for the identification of methods for expressing heterologous polypeptides in microalgae as well as alternative compositions for therapeutic applications.

### BRIEF SUMMARY OF THE INVENTION

The present invention is defined in the attached claims.

The present invention provides a method for production of a viral neuraminidase (NA) protein, comprising:
culturing a recombinant microalgal cell in a medium such that the NA protein is secreted into the medium, wherein the recombinant microalgal cell comprises a nucleic acid molecule comprising a polynucleotide sequence that encodes the viral NA protein, wherein
the viral NA protein comprises a membrane domain; and
recovering the secreted recombinant viral NA protein from the medium.

In some embodiments, the NA protein is at least 90% identical to SEQ ID NO: 100.

In some embodiments, the microalgal cell is a member of the order Thraustochytriales. In some embodiments, the microalgal cell is a *Schizochytrium* or a *Thraustochytrium.* In some embodiments, the polynucleotide sequence encoding the viral protein further comprises a HA membrane domain. In some embodiments, the nucleic acid molecule further comprises a polynucleotide sequence selected from the group consisting of: SEQ ID NO: 2, SEQ ID NO: 3, SEQ ID NO: 4, SEQ ID NO: 38, SEQ ID NO: 42, SEQ ID NO: 43, SEQ ID NO: 44, SEQ ID NO: 45, SEQ ID NO: 46, and combinations thereof.

The methods of the present invention can be used to produce an isolated viral NA protein.

Also disclosed herein but not claimed as such is a recombinant microalgal cell comprising a nucleic acid molecule comprising a polynucleotide sequence that encodes a viral protein selected from the group consisting of a hemagglutinin (HA) protein, a neuraminidase (NA) protein, a fusion (F) protein, a glycoprotein (G) protein, an envelope (E) protein, a glycoprotein of 120 kDa (gp120), a glycoprotein of 41 kDa (gp41), a matrix protein, and combinations thereof. In some instances of the disclosure, the viral protein is a HA protein. In some instances, the HA protein is at least 90% identical to SEQ ID NO: 77. In some instances, the microalgal cell is capable of post-translational processing of the HA protein to produce HA1 and HA2 polypeptides in the absence of exogenous protease. In some instances, the viral protein is a NA protein. In some instances, the NA protein is at least 90% identical to SEQ ID NO: 100. In some instances, the viral protein is a F protein. In some instances, the F protein is at least 90% identical to SEQ ID NO: 102. In some instances, the viral protein is a G protein. In some instances, the G protein is at least 90% identical to SEQ ID NO: 103. In some instances, the microalgal cell is a member of the order Thraustochytriales. In some instances, the microalgal cell is a *Schizochytrium* or a *Thraustochytrium.* In some instances, the polynucleotide sequence encoding the viral protein further comprises a HA membrane domain. In some instances, the nucleic acid molecule further comprises a polynucleotide sequence selected from the group consisting of: SEQ ID NO: 2, SEQ ID NO: 3, SEQ ID NO: 4, SEQ ID NO: 38, SEQ ID NO: 42, SEQ ID NO: 43, SEQ ID NO: 44, SEQ ID NO: 45, SEQ ID NO: 46, and combinations thereof.

The methods of the present invention can be used to produce a composition comprising a microalgal extracellular body and a viral NA protein comprising a membrane domain.

In some embodiments, the method further comprises removing the culture supernatant from the composition and resuspending the extracellular body in an aqueous liquid carrier.

In some embodiments, the method comprises a host cell that is a *Labyrinthulomycota* host cell. In some embodiments, the host cell is a *Schizochytrium* or *Thraustochytrium* host cell.

### BRIEF DESCRIPTION OF THE DRAWINGS

**FIG. 1** shows the polynucleotide sequence (SEQ ID NO: 76) that encodes the hemagglutinin (HA) protein of influenza A virus (A/Puerto Rico/8/34/Mount Sinai(H1N1)), which has been codon-optimized for expression in *Schizochytrium* sp. ATCC 20888.
**FIG. 2** shows a plasmid map of pCL0143.
**FIG. 3** shows the procedure used for the analysis of the CL0143-9 clone.
**FIG. 4** shows secretion of HA protein by transgenic *Schizochytrium* CL0143-9 ("E"). **FIG. 4A** shows the recovered recombinant HA protein (as indicated by arrows) in anti-H1N1 immunoblots from the low-speed supernatant (*i.e.,* cell-free supernatant ("CFS")) of cultures at various temperatures (25°C, 27°C, 29°C) and pH (5.5, 6.0, 6.5, 7.0). **FIG. 4B** shows the recovered recombinant HA protein in Coomassie stained gels ("Coomassie") and anti-H1N1 immunoblots ("IB: anti-H1N1") from the 60% sucrose fraction under non-reducing or reducing conditions.

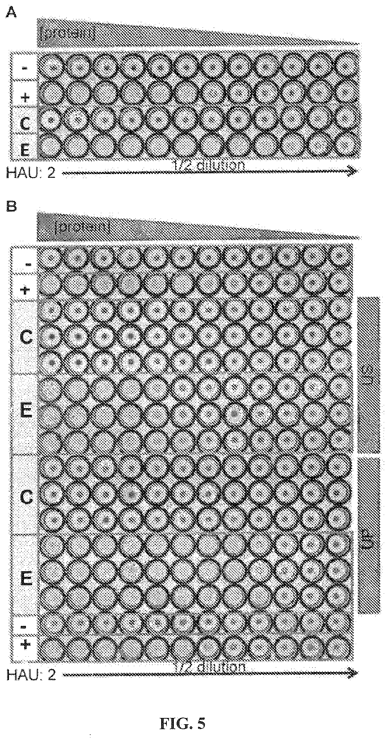

**FIG. 5** shows hemagglutination activity of recombinant HA protein from transgenic *Schizochytrium* CL0143-9 ("E"). **FIG. 5A** shows hemagglutination activity in cell-free supernatant ("CFS"). **FIG. 5B** shows hemagglutination activity in soluble ("US") and insoluble ("UP") fractions. "[protein]" refers to the concentration of protein, decreasing from left to right with increasing dilutions of the samples. "-" refers to negative control lacking HA. "+" refers to Influenza hemagglutinin positive control. "C" refers to the negative control wild-type strain of *Schizochytrium* sp. ATCC 20888. "HAU" refers to Hemagglutinin Activity Unit based on the fold dilution of samples from left to right. "2" refers to a two-fold dilution of the sample in the first well; subsequent wells from left to right represent doubling dilutions over the previous well, such that the fold dilutions from the first to last wells from left to right were 2, 4, 8, 16, 32, 64, 128, 256, 512, 1024, 2048, and 4096.

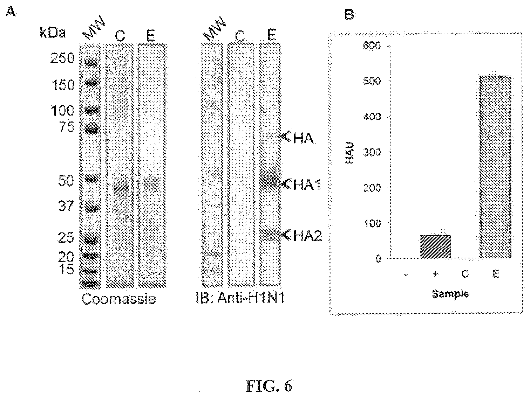

**FIG. 6** shows the expression and hemagglutination activity of HA protein present in the 60% sucrose fraction for transgenic *Schizochytrium* CL0143-9 ("E"). **FIG. 6A** shows the recovered recombinant HA protein (as indicated by arrows) in the Coomassie stained gel ("Coomassie") and anti-H1N1 immunoblot ("IB: anti-H1N1") from the 60% sucrose fraction. **FIG. 6B** shows the corresponding hemagglutination activity. "-" refers to negative control lacking HA. "+" refers to Influenza HA protein positive control. "C" refers to the negative control wild-type strain of *Schizochytrium* sp. ATCC 20888. "HAU" refers to Hemagglutinin Activity Unit based on the fold dilution of samples.
**FIG. 7** shows peptide sequence analysis for the recovered recombinant HA protein, which was identified by a total of 27 peptides (the amino acids associated with the peptides are highlighted in **bold** font), covering over 42% of the entire HA protein sequence (SEQ ID NO: 77). The HA1 polypeptide was identified by a total of 17 peptides, and the HA2 polypeptide was identified by a total of 9 peptides.
**FIG. 8** shows a Coomassie stained gel ("Coomassie") and anti-H1N1 immunoblot ("IB: anti-H1N1") illustrating HA protein glycosylation in *Schizochytrium.* "EndoH" and "PNGase F" refer to enzymatic treatments of the 60% sucrose fraction of transgenic *Schizochytrium* CL0143-9 with the respective enzymes. "NT" refers to transgenic *Schizochytrium* CL0143-9 incubated without enzymes but under the same conditions as the EndoH and PNGase F treatments.
**FIG. 9** shows total *Schizochytrium* sp. ATCC 20888 culture supernatant protein (g/L) over time (hours).
**FIG. 10** shows an SDS-PAGE of total *Schizochytrium* sp. ATCC 20888 culture supernatant protein in lanes 11-15, where the supernatant was collected at five of the six timepoints shown in **FIG. 9** for hours 37-68, excluding hour 52. Bands identified as Actin and Gelsolin (by mass spectral peptide sequencing) are marked with arrows. Lane 11 was loaded with 2.4 µg of total protein; the remaining wells were loaded with 5 µg total protein.
**FIG. 11** shows negatively-stained vesicles from *Schizochytrium* sp. ATCC 20888 ("C: 20888") and transgenic *Schizochytrium* CL0143-9 ("E: CL0143-9").
**FIG. 12** shows anti-H1N1 immunogold labeled vesicles from *Schizochytrium* sp. ATCC 20888 ("C: 20888") and transgenic *Schizochytrium* CL0143-9 ("E: CL0143-9").
**FIG. 13** shows predicted signal anchor sequences native to *Schizochytrium* based on use of the SignalP algorithm. *See,* e.g., Bendsten et al., J, Mol. Biol. 340: 783-795 (2004); Nielsen, H. and Krogh, A. Proc. Int. Conf. Intell. Syst. Mol. Biol. 6: 122-130 (1998); Nielsen, H., et al., Protein Engineering 12: 3-9 (1999); Emanuelsson, O. et al., Nature Protocols 2: 953-971 (2007).
**FIG. 14** shows predicted Type I membrane proteins in *Schizochytrium* based on BLAST searches of genomic and EST DNA *Schizochytrium* databases for genes with homology to known Type I membrane proteins from other organisms and having membrane spanning regions in the extreme C-terminal region of the proteins. Putative membrane spanning regions are shown in bold font.
**FIG. 15** shows a plasmid map of pCL0120.
**FIG. 16** shows a codon usage table for *Schizochytrium.*
**FIG. 17** shows a plasmid map of pCL0130.
**FIG. 18** shows a plasmid map of pCL0131.
**FIG. 19** shows a plasmid map of pCL0121.
**FIG. 20** shows a plasmid map of pCL0122.
**FIG. 21** shows the polynucleotide sequence (SEQ ID NO: 92) that encodes the *Piromyces* sp. E2 xylose isomerase protein "XylA", corresponding to GenBank Accession number CAB76571, optimized for expression in *Schizochytrium* sp. ATCC 20888.
**FIG. 22** shows the polynucleotide sequence (SEQ ID NO: 93) that encodes the *Piromyces* sp. E2 xylulose kinase protein "XylB", corresponding to GenBank Accession number AJ249910, optimized for expression in *Schizochytrium* sp. ATCC 20888.
**FIG. 23** shows a plasmid map of pCL0132.
**FIG. 24** shows a plasmid map of pCL0136.
**FIG. 25A** shows a plasmid map of pCL0140 and **FIG. 25B** shows a plasmid map ofpCL0149.
**FIG. 26A** shows the polynucleotide sequence (SEQ ID NO: 100) that encodes neuraminidase (NA) protein of influenza A virus (A/Puerto Rico/8/34/Mount Sinai (H1N1)), optimized for expression in *Schizochytrium* sp. ATCC 20888. **FIG. 26B** shows the polynucleotide sequence (SEQ ID NO: 101) that encodes NA protein of influenza A virus (A/Puerto Rico/8/34/Mount Sinai (H1N1)) followed by a V5 tag and a polyhistidine tag, optimized for expression in *Schizochytrium* sp. ATCC 20888.
**FIG. 27** shows a scheme of the procedure used for the analysis of the CL0140 and CL0149 clones.

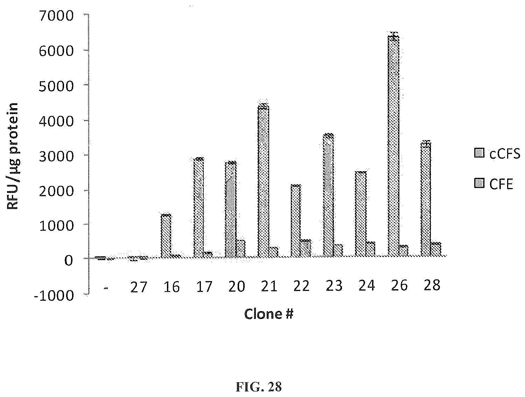

**FIG. 28** shows neuraminidase activity of recombinant NA from transgenic *Schizochytrium* strains CL0140-16, -17, -20, -21, -22, -23, -24, -26, -28. Activity is determined by measuring the fluorescence of 4-methylumbelliferone which arises following the hydrolysis of 4-Methylumbelliferyl -α-D-*N*-Acetylneuraminate (4-MUNANA) by sialidases (Excitation (Exc): 365nm, Emission (Em): 450nm). Activity is expressed as relative fluorescence units (RFU) per µg protein in the concentrated cell-free supernatant (cCFS, leftmost bar for each clone) and the cell-free extract (CFE, rightmost bar for each clone). The wild-type strain of *Schizochytrium* sp. ATCC 20888 ("-") and a PCR-negative strain of *Schizochytrium* transformed with pCL0140 ("27"), grown and prepared in the same manner as the transgenic strains, were used as negative controls.

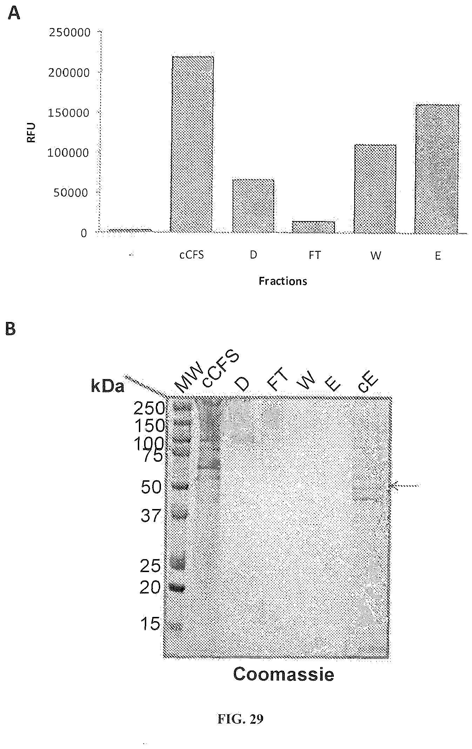

**FIG. 29** shows partial purification of the recombinant NA protein from transgenic *Schizochytrium* strain CL0140-26. The neuraminidase activity of the various fractions is shown in **FIG. 29A****.** "cCFS" refers to the concentrated cell-free supernatant. "D" refers to the cCFS diluted with washing buffer, "FT" refers to the flow-through, "W" refers to the wash, "E" refers to the elute and "cE" refers to the concentrated elute fraction. The Coomassie stained gel ("Coomassie") of 12.5 µL of each fraction is shown in **FIG. 29B****.** The arrow points to the band identified as the NA protein. SDS-PAGE was used to separate the proteins on NuPAGE^{®} Novex^{®} 12% bis-tris gels with MOPS SDS running buffer.
**FIG. 30** shows peptide sequence analysis for the recovered recombinant NA protein, which was identified by a total of 9 peptides (highlighted in bold red), covering 25% of the protein sequence (SEQ ID NO: 100).

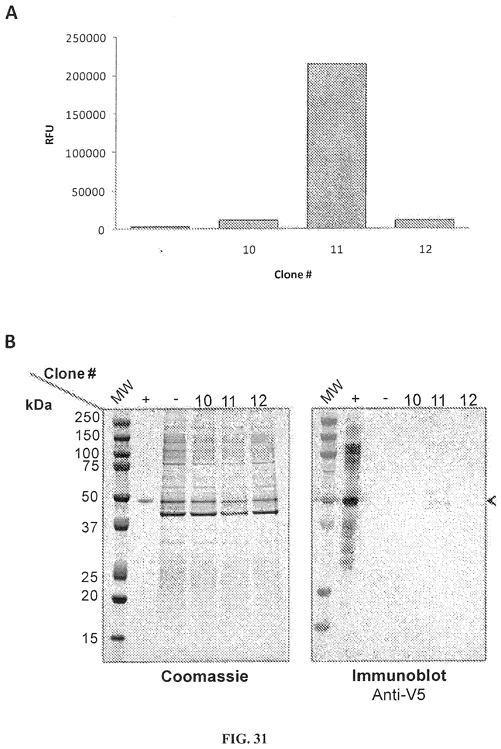

**FIG. 31** shows the neuraminidase activities of transgenic *Schizochytrium* strains CL0149-10, -11, -12 and corresponding Coomassie stained gel ("Coomassie") and anti-V5 immunoblot (("Immunoblot: anti-V5"). **FIG. 31A** shows neuraminidase activity, as determined by measuring the fluorescence of 4-methylumbelliferone which arises following the hydrolysis of 4-MUNANA by sialidases (Exc: 365nm, Em: 450nm). Activity is expressed as relative fluorescence units (RFU) per µg protein in the cell-free supernatant (CFS). The wild-type strain of *Schizochytrium* sp. ATCC 20888 ("-"), grown and prepared in the same manner as the transgenic strain, was used as negative control. **FIG. 31B** shows the Coomassie stained gel and corresponding anti-V5 immunoblot on 12.5 µL CFS for 3 transgenic *Schizochytrium* CL0149 strains ("10", "11", and "12"). The Positope^{™} antibody control protein was used as a positive control ("+"). The wild-type strain of *Schizochytrium* sp. ATCC 20888 ("-"), grown and prepared in the same manner as the transgenic strain, was used as negative control.

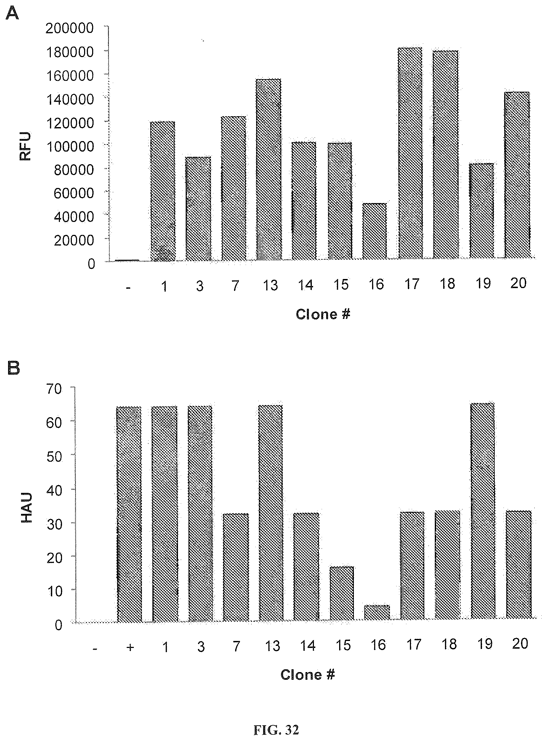

**FIG. 32** shows the enzymatic activities of Influenza HA and NA in the cell-free supernatant of transgenic *Schizochytrium* cotransformed with CL0140 and CL0143. Data are presented for clones CL0140-143-1, -3, -7, -13, -14, -15, -16, -17, -18, -19, -20. **FIG.** 32A shows the neuraminidase activity, as determined by measuring the fluorescence of 4-methylumbelliferone which arises following the hydrolysis of 4-MUNANA by sialidases (Exc: 365nm, Em: 450nm). Activity is expressed as relative fluorescence units (RFU) in 25 µL CFS. The wild-type strain of *Schizochytrium* sp. ATCC 20888 ("-"), grown and prepared in the same manner as the transgenic strain, was used as negative control. **FIG. 32B** shows the hemagglutination activity. "-" refers to negative control lacking HA. "+" refers to Influenza HA positive control. "HAU" refers to Hemagglutinin Activity Unit based on the fold dilution of samples.

### DETAILED DESCRIPTION

The technical information set out below may in some respects go beyond the scope of the invention, which is defined exclusively by the appended claims. The additional technical information is provided to place the actual invention in a broader technical context and to illustrate possible related technical developments and to aid understanding of the invention. Any additional technical information which does not fall within the scope of the appended claims is not as such claimed or to be construed as being part of the invention. The present invention is directed to methods for production of a viral neuraminidase (NA) protein as defined in the attached claims.

### Microalgal Host Cells

Microalgae, also known as microscopic algae, are often found in freshwater and marine systems. Microalgae are unicellular but can also grow in chains and groups. Individual cells range in size from a few micrometers to a few hundred micrometers. Because the cells are capable of growing in aqueous suspensions, they have efficient access to nutrients and the aqueous environment.

In some embodiments, the microalgal host cell is a heterokont or stramenopile.

In some embodiments, the microalgal host cell is a member of the phylum Labyrinthulomycota. In some embodiments, the Labyrinthulomycota host cell is a member of the order Thraustochytriales or the order Labyrinthulales. According to the present invention, the term "thraustochytrid" refers to any member of the order Thraustochytriales, which includes the family Thraustochytriaceae, and the term "labyrinthulid" refers to any member of the order Labyrinthulales, which includes the family Labyrinthulaceae. Members of the family Labyrinthulaceae were previously considered to be members of the order Thraustochytriales, but in more recent revisions of the taxonomic classification of such organisms, the family Labyrinthulaceae is now considered to be a member of the order Labyrinthulales. Both Labyrinthulales and Thraustochytriales are considered to be members of the phylum Labyrinthulomycota. Taxonomic theorists now generally place both of these groups of microorganisms with the algae or algae-like protists of the Stramenopile lineage. The current taxonomic placement of the thraustochytrids and labyrinthulids can be summarized as follows:
Realm: Stramenopila (Chromista)
Phylum: Labyrinthulomycota (Heterokonta)
Class: Labyrinthulomycetes (Labyrinthulae)
Order: Labyrinthulales
   Family: Labyrinthulaceae
Order: Thraustochytriales
   Family: Thraustochytriaceae

For purposes of the present invention, strains described as thraustochytrids include the following organisms: Order: Thraustochytriales; Family: Thraustochytriaceae; Genera: *Thraustochytrium* (Species: sp., *arudimentale, aureum, benthicolα, globosum, kinnei, motivum, multirudimentale, pachydermum, proliferum, roseum, striatum*), *Ulkenia* (Species: sp., *amoeboidea, kerguelensis, minuta, profunda, radiata, sailens, sarkariana, schizochytrops, visurgensis, yorkensis), Schizochytrium* (Species: sp., *aggregatum, limnaceum, mangrovei, minutum, octosporum), Japonochytrium* (Species: sp., *marinum*), *Aplanochytrium* (Species: sp., *haliotidis, kerguelensis, profunda, stocchinoi*), *Althornia* (Species: sp., *crouchii*), or *Elina* (Species: sp., *marisalba, sinorifica*)*.* For the purposes of this invention, species described within *Ulkenia* will be considered to be members of the genus *Thraustochytrium. Aurantiochytrium, Oblongichytrium, Botryochytrium, Parietichytrium,* and *Sicyoidochytrium* are additional genuses encompassed by the phylum Labyrinthulomycota in the present invention.

Strains described in the present invention as Labyrinthulids include the following organisms: Order: Labyrinthulales, Family: Labyrinthulaceae, Genera: *Labyrinthula* (Species: sp., *algeriensis, coenocystis, chattonii, macrocystis, macrocystis atlantica, macrocystis macrocystis, marina, minuta, roscoffensis, valkanovii, vitellina, vitellina pacifica, vitellina vitellina, zopfii*), *Labyrinthuloides* (Species: sp., *haliotidis, yorkensis*), *Labyrinthomyxa* (Species: sp., *marina*), *Diplophrys* (Species: sp., *archeri*), *Pyrrhosorus* (Species: sp., *marinus*), *Sorodiplophrys* (Species: sp., *stercorea*) or *Chlamydomyxa* (Species: sp., *labyrinthuloides, montana*) (although there is currently not a consensus on the exact taxonomic placement of *Pyrrhosorus, Sorodiplophrys* or *Chlamydomyxa*)*.*

Microalgal cells of the phylum Labyrinthulomycota include, but are not limited to, deposited strains PTA-10212, PTA-10213, PTA-10214, PTA-10215, PTA-9695, PTA-9696, PTA-9697, PTA-9698, PTA-10208, PTA-10209, PTA-10210, PTA-10211, the microorganism deposited as SAM2179 (named "Ulkenia SAM2179" by the depositor), any *Thraustochytrium* species (including former *Ulkenia* species such as *U. visurgensis, U. amoeboida, U. sarkariana, U. profunda, U radiata, U. minuta and Ulkenia* sp. BP-5601), and including *Thraustochytrium striatum, Thraustochytrium aureum, Thraustochytrium roseum;* and any *Japonochytrium* species. Strains of *Thraustochytriales* include, but are not limited to *Thraustochytrium* sp. (23B) (ATCC 20891); *Thraustochytrium striatum* (Schneider) (ATCC 24473); *Thraustochytrium aureum* (Goldstein) (ATCC 34304); *Thraustochytrium roseum* (Goldstein) (ATCC 28210); and *Japonochytrium* sp. (L1) (ATCC 28207). *Schizochytrium* include, but are not limited to *Schizochytrium aggregatum, Schizochytrium limacinum, Schizochytrium* sp. (S31) (ATCC 20888), *Schizochytrium* sp. (S8) (ATCC 20889), *Schizochytrium* sp. (LC-RM) (ATCC 18915), *Schizochytrium* sp. (SR 21), deposited strain ATCC 28209, and deposited *Schizochytrium limacinum* strain IFO 32693. In some embodiments, the cell is a *Schizochytrium* or a *Thraustochytrium. Schizochytrium* can replicate both by successive bipartition and by forming sporangia, which ultimately release zoospores. *Thraustochytrium,* however, replicate only by forming sporangia, which then release zoospores.

In some embodiments, the microalgal host cell is a Labyrinthulae (also termed Labyrinthulomycetes). Labyrinthulae produce unique structures called "ectoplasmic nets." These structures are branched, tubular extensions of the plasma membrane that contribute significantly to the increased surface area of the plasma membrane. *See,* for example, Perkins, Arch. Mikrobiol. 84:95-118 (1972); Perkins, Can. J. Bot. 57:485-491 (1973). Ectoplasmic nets are formed from a unique cellular structure referred to as a sagenosome or bothrosome. The ectoplasmic net attaches Labyrinthulae cells to surfaces and is capable of penetrating surfaces. *See,* for example, Coleman and Vestal, Can. J. Microbiol. 33:841-843 (1987), and Porter, Mycologia 84:298-299 (1992), respectively. *Schizochytrium* sp. ATCC 20888, for example, has been observed to produce ectoplasmic nets extending into agar when grown on solid media (data not shown). The ectoplasmic net in such instances appears to act as a pseudorhizoid. Additionally, actin filaments have been found to be abundant within certain ectoplasmic net membrane extensions. *See,* for example, Preston, J. *Eukaryot. Microbiol. 52*:461-475 (2005). Based on the importance of actin filaments within cytoskeletal structures in other organisms, it is expected that cytoskeletal elements such as actin play a role in the formation and/or integrity of ectoplasmic net membrane extensions.

Additional organisms producing pseudorhizoid extensions include organisms termed chytrids, which are taxonomically classified in various groups including the Chytridiomycota, or Phycomyces. Examples of genera include Chytrdium, Chytrimyces, Cladochytium, Lacustromyces, Rhizophydium, Rhisophyctidaceae, Rozella, Olpidium, and Lobulomyces.

In some embodiments, the microalgal host cell comprises a membrane extension. In some embodiments, the microalgal host cell comprises a pseudorhizoid. In some embodiments, the microalgal host cell comprises an ectoplasmic net. In some embodiments, the microalgal host cell comprises a sagenosome or bothrosome.

In some embodiments, the microalgal host cell is a thraustochytrid. In some embodiments, the microalgal host cell is a *Schizochytrium* or *Thraustochytrium* cell.

In some embodiments, the microalgal host cell is a labyrinthulid.

In some embodiments, the microalgal host cell is a eukaryote capable of processing polypeptides through a conventional secretory pathway, such as members of the phylum Labyrinthulomycota, including *Schizochytrium, Thraustochytrium,* and other thraustochytrids. For example, it has been recognized that members of the phylum Labyrinthulomycota produce fewer abundantly-secreted proteins than CHO cells, resulting in an advantage of using *Schizochytrium,* for example, over CHO cells. In addition, unlike *E. coli,* members of the phylum Labyrinthulomycota, such as *Schizochytrium,* perform protein glycosylation, such as N-linked glycosylation, which is required for the biological activity of certain proteins. It has been determined that the N-linked glycosylation exhibited by thraustochytrids such as *Schizochytrium* more closely resembles mammalian glycosylation patterns than does yeast glycosylation.

Effective culture conditions for the host cell include, but are not limited to, effective media, bioreactor, temperature, pH, and oxygen conditions that permit protein production and/or recombination. An effective medium refers to any medium in which a microalgal cell, such as a Thraustochytriales cell, e.g., a *Schizochytrium* host cell, is typically cultured. Such medium typically comprises an aqueous medium having assimilable carbon, nitrogen, and phosphate sources, as well as appropriate salts, minerals, metals, and other nutrients, such as vitamins. Non-limiting examples of suitable media and culture conditions are disclosed in the Examples section. Non-limiting culture conditions suitable for Thraustochytriales microorganisms are also described in U.S. Patent No. 5,340,742 .

Cells can be cultured in conventional fermentation bioreactors, shake flasks, test tubes, microtiter dishes, and petri plates. Culturing can be carried out at a temperature, pH, and oxygen content appropriate for a recombinant cell.

In some embodiments, the microalgal host cell contains a recombinant vector comprising a nucleic acid sequence encoding a selection marker. In some embodiments, the selection marker allows for the selection of transformed microorganisms. Examples of dominant selection markers include enzymes that degrade compounds with antibiotic or fungicide activity such as, for example, the Sh ble gene from *Steptoalloteichus hindustanus,* which encodes a "bleomycin-binding protein" represented by SEQ ID NO:5. Another example of a dominant selection marker includes a thraustochytrid acetolactate synthase sequence such as a mutated version of the polynucleotide sequence of SEQ ID NO:6. The acetolactate synthase can be modified, mutated, or otherwise selected to be resistant to inhibition by sulfonylurea compounds, imidazolinone-class inhibitors, and/or pyrimidinyl oxybenzoates. Representative examples of thraustochytrid acetolactate synthase sequences include, but are not limited to, amino acid sequences such as SEQ ID NO:7, SEQ ID NO:8, SEQ ID NO:9, SEQ ID NO:10, or an amino acid sequence that differs from SEQ ID NO:7 by an amino acid deletion, insertion, or substitution at one or more of the following positions: 116G, 117A, 192P, 200A, 251K, 358M, 383D, 592V, 595W, or 599F, and polynucleotide sequences such as SEQ ID NO:11, SEQ ID NO:12, or SEQ ID NO:13, as well as sequences having at least 90%, at least 95%, at least 96%, at least 97%, at least 98%, or at least 99% identity to any of the representative sequences. Further examples of selection markers that can be contained in a recombinant vector for transformation of microalgal cells include ZEOCIN^{™}, paromomycin, hygromycin, blasticidin, or any other appropriate resistance marker.

The term "transformation" is used to refer to any method by which an exogenous nucleic acid molecule (i.e., a recombinant nucleic acid molecule) can be inserted into microbial cells. In microbial systems, the term "transformation" is used to describe an inherited change due to the acquisition of exogenous nucleic acids by the microorganism and is essentially synonymous with the term "transfection." Suitable transformation techniques for introducing exogenous nucleic acid molecules into the microalgal host cells include, but are not limited to, particle bombardment, electroporation, microinjection, lipofection, adsorption, infection, and protoplast fusion. For example, exogenous nucleic acid molecules, including recombinant vectors, can be introduced into a microalgal cell that is in a stationary phase during the exponential growth phase, or when the microalgal cell reaches an optical density of 1.5 to 2 at 600 nm. A microalgal host cell can also be pretreated with an enzyme having protease activity prior to introduction of a nucleic acid molecule into the host cell by electroporation.

In some embodiments, the host cell can be genetically modified to introduce or delete genes involved in biosynthetic pathways associated with the transport and/or synthesis of carbohydrates, including those involved in glycosylation. For example, the host cell can be modified by deleting endogenous glycosylation genes and/or inserting human or animal glycosylation genes to allow for glycosylation patterns that more closely resemble those of humans. Modification of glycosylation in yeast can be found, for example, in U.S. Patent No. 7,029,872 and U.S. Publ. Nos. 2004/0171826, 2004/0230042, 2006/0257399, 2006/0029604, and 2006/0040353. A host cell
also includes a cell in which an RNA viral element is employed to increase or regulate gene expression.

### Expression Systems

The expression system used for expression of a heterologous polypeptide in a microalgal host cell comprises regulatory control elements that are active in microalgal cells. In some embodiments, the expression system comprises regulatory control elements that are active in Labyrinthulomycota cells. In some embodiments, the expression system comprises regulatory control elements that are active in thraustochytrids. In some embodiments, the expression system comprises regulatory control elements that are active in *Schizochytrium* or *Thraustochytrium.* Many regulatory control elements, including various promoters, are active in a number of diverse species. Therefore, regulatory sequences can be utilized in a cell type that is identical to the cell from which they were isolated or can be utilized in a cell type that is different than the cell from which they were isolated. The design and construction of such expression cassettes use standard molecular biology techniques known to persons skilled in the art. *See,* for example, Sambrook et al., 2001, Molecular Cloning: A Laboratory Manual, 3rd edition.

In some embodiments, the expression system used for heterologous polypeptide production in microalgal cells comprises regulatory elements that are derived from Labyrinthulomycota sequences. In some embodiments, the expression system used to produce heterologous polypeptides in microalgal cells comprises regulatory elements that are derived from non-Labyrinthulomycota sequences, including sequences derived from non-Labyrinthulomycota algal sequences. In some embodiments, the expression system comprises a polynucleotide sequence encoding a heterologous polypeptide, wherein the polynucleotide sequence is associated with any promoter sequence, any terminator sequence, and/or any other regulatory sequences that are functional in a microalgal host cell. Inducible or constitutively active sequences can be used. Suitable regulatory control elements also include any of the regulatory control elements associated with the nucleic acid molecules described herein.

The methods of the present invention may utilise an expression cassette for expression of a heterologous polypeptide (viral NA protein comprising a membrane domain) in a microalgal host cell. The methods of the present invention may also utilise any of the above-described host cells comprising an expression cassette for expression of the heterologous polypeptide in the host cell. In some embodiments, the expression system comprises an expression cassette containing genetic elements, such as at least a promoter, a coding sequence, and a terminator region operably linked in such a way that they are functional in a host cell. In some embodiments, the expression cassette comprises at least one of the isolated nucleic acid molecules as described herein. In some embodiments, all of the genetic elements of the expression cassette are sequences associated with isolated nucleic acid molecules. In some embodiments, the control sequences are inducible sequences. In some embodiments, the nucleic acid sequence encoding the heterologous polypeptide is integrated into the genome of the host cell. In some embodiments, the nucleic acid sequence encoding the heterologous polypeptide is stably integrated into the genome of the host cell.

In some embodiments, an isolated nucleic acid sequence encoding the heterologous polypeptide to be expressed is operably linked to a promoter sequence and/or a terminator sequence, both of which are functional in the host cell. The promoter and/or terminator sequence to which the isolated nucleic acid sequence encoding the heterologous polypeptide to be expressed is operably linked can include any promoter and/or terminator sequence, including but not limited to the nucleic acid sequences disclosed herein, the regulatory sequences disclosed in U.S. Patent No. 7,001,772, the regulatory sequences disclosed in U.S. Publ. Nos. 2006/0275904 and 2006/0286650, the regulatory sequence disclosed in U.S. Publ. No. 2010/0233760 and WO 2010/107709, or other regulatory sequences functional in the host cell in which they are transformed that are operably linked to the isolated polynucleotide sequence encoding a heterologous polypeptide. In some embodiments, the nucleic acid sequence encoding the heterologous polypeptide is codon-optimized for the specific microalgal host cell to maximize translation efficiency. Again, in the invention, the heterologous polypeptide is a viral NA protein comprising a membrane domain.

The methods of the present invention may utilise recombinant vectors comprising an expression cassette. Recombinant vectors include, but are not limited to, plasmids, phages, and viruses. In some embodiments, the recombinant vector is a linearized vector. In some embodiments, the recombinant vector is an expression vector. As used herein, the phrase "expression vector" refers to a vector that is suitable for production of an encoded product (e.g., a protein of interest). In some embodiments, a nucleic acid sequence encoding the product to be produced is inserted into the recombinant vector to produce a recombinant nucleic acid molecule. The nucleic acid sequence encoding the heterologous polypeptide to be produced is inserted into the vector in a manner that operatively links the nucleic acid sequence to regulatory sequences in the vector (e.g., a Thraustochytriales promoter), which enables the transcription and translation of the nucleic acid sequence within the recombinant microorganism. In some embodiments, a selectable marker, including any of the selectable markers described herein, enables the selection of a recombinant microorganism into which a recombinant nucleic acid molecule has successfully been introduced.

In some embodiments, the heterologous viral NA protein produced by the host cell
is produced at commercial scale. Commercial scale includes production of heterologous polypeptide from a microorganism grown in an aerated fermentor of a size ≥100 L, ≥1,000 L, ≥10,000 L or ≥100,000 L. In some embodiments, the commercial scale production is done in an aerated fermentor with agitation.

In the invention, a viral NA protein produced by a host cell is secreted from the cell into the culture medium.

In the invention, the viral NA protein is recovered from the culture medium, or fermentation medium in which the cell is grown. In some embodiments, the heterologous polypeptide
is recovered from the culture media as a soluble heterologous polypeptide. In some embodiments, the heterologous polypeptide is a secreted protein comprising a signal peptide.

In some embodiments, the heterologous polypeptide (viral NA protein) produced by the invention comprises a targeting signal directing its extracellular secretion. In some embodiments, the heterologous polypeptide comprises a signal peptide. In some embodiments, the heterologous polypeptide comprises a Na/Pi-IIb2 transporter signal peptide or Sec1 transport protein. In some embodiments, the signal peptide comprises the amino acid sequence of SEQ ID NO:1 or SEQ ID NO:37. In some embodiments, the heterologous polypeptide comprising a signal peptide having the amino acid sequence of SEQ ID NO:1 or SEQ ID NO:37 is secreted into the culture medium. In some embodiments, the signal peptide is cleaved from the protein during the secretory process, resulting in a mature form of the protein.

In some embodiments, the heterologous polypeptide (viral NA protein) produced a host cell
is glycosylated. In some embodiments, the glycosylation pattern of the heterologous polypeptide produced by the invention more closely resembles mammalian glycosylation patterns than proteins produced in yeast or *E. coli.* In some embodiments, the heterologous polypeptide produced by a microalgal host cell
comprises a N-linked glycosylation pattern. Glycosylated proteins used for therapeutic purposes are less likely to promote anti-glycoform immune responses when their glycosylation patterns are similar to glycosylation patterns found in a subject organism. Conversely, glycosylated proteins having linkages or sugars that are not characteristic of a subject organism are more likely to be antigenic. Effector functions can also be modulated by specific glycoforms. For example, IgG can mediate pro- or antiinflammatory reactions in correlation with the absence or presence, respectively, of terminal sialic acids on Fc region glycoforms (Kaneko et al., Science 313:670-3 (2006)).

The method of producing a recombinant heterologous polypeptide (viral NA protein comprising a membrane domain) comprises culturing a recombinant microalgal host cell under conditions sufficient to express a polynucleotide sequence encoding the heterologous polypeptide. The recombinant heterologous polypeptide is secreted from the host cell and is recovered from the culture medium. In some embodiments, a heterologous polypeptide that is secreted from the cell comprises a secretion signal peptide.

As used herein, the phrase "recovering the protein" refers to collecting fermentation medium containing the protein and need not imply additional steps of separation or purification. Heterologous polypeptides produced by the method of the present invention can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing, and differential solubilization. In some embodiments, heterologous polypeptides produced by the method of the present invention are isolated in "substantially pure" form. As used herein, "substantially pure" refers to a purity that allows for the effective use of the heterologous polypeptide as a commercial product.

In some embodiments, the host cell of the method is a thraustochytrid. In some embodiments, the host cell of the method is a *Schizochytrium* or a *Thraustochytrium.*

In some embodiments, a recombinant vector used in the methods of the invention is a targeting vector. As used herein, the phrase "targeting vector" refers to a vector that is used to deliver a particular nucleic acid molecule into a recombinant cell, wherein the nucleic acid molecule is used to delete or inactivate an endogenous gene within the host cell (i.e., used for targeted gene disruption or knock-out technology). Such a vector is also known as a "knock-out" vector. In some embodiments, a portion of the targeting vector has a nucleic acid sequence that is homologous to a nucleic acid sequence of a target gene in the host cell (i.e., a gene which is targeted to be deleted or inactivated). In some embodiments, the nucleic acid molecule inserted into the vector (i.e., the insert) is homologous to the target gene. In some embodiments, the nucleic acid sequence of the vector insert is designed to bind to the target gene such that the target gene and the insert undergo homologous recombination, whereby the endogenous target gene is deleted, inactivated, or attenuated (i.e., by at least a portion of the endogenous target gene being mutated or deleted).

### Isolated Nucleic Acid Molecules

An isolated nucleic acid molecule that has utility in the methods of the present invention is a nucleic acid molecule that has been removed from its natural milieu (i.e., that has been subject to human manipulation), its natural milieu being the genome or chromosome in which the nucleic acid molecule is found in nature. As such, "isolated" does not necessarily reflect the extent to which the nucleic acid molecule has been purified, but indicates that the molecule does not include an entire genome or an entire chromosome in which the nucleic acid molecule is found in nature. An isolated nucleic acid molecule can include DNA, RNA (e.g., mRNA), or derivatives of either DNA or RNA (e.g., cDNA). Although the phrase "nucleic acid molecule" primarily refers to the physical nucleic acid molecule and the phrases "nucleic acid sequence" or "polynucleotide sequence" primarily refers to the sequence of nucleotides on the nucleic acid molecule, the phrases are used interchangeably, especially with respect to a nucleic acid molecule, polynucleotide sequence, or a nucleic acid sequence that is capable of encoding a heterologous polypeptide. In some embodiments, an isolated nucleic acid molecule
is produced using recombinant DNA technology (e.g., polymerase chain reaction (PCR) amplification, cloning) or chemical synthesis. Isolated nucleic acid molecules include natural nucleic acid molecules and homologues thereof, including, but not limited to, natural allelic variants and modified nucleic acid molecules in which nucleotides have been inserted, deleted, substituted, and/or inverted in such a manner that such modifications provide the desired effect on sequence, function, and/or the biological activity of the encoded heterologous polypeptide.

A nucleic acid sequence complement of a promoter sequence, terminator sequence, signal peptide sequence, or any other sequence refers to the nucleic acid sequence of the nucleic acid strand that is complementary to the strand with the promoter sequence, terminator sequence, signal peptide sequence, or any other sequence. It will be appreciated that a double-stranded DNA comprises a single-strand DNA and its complementary strand having a sequence that is a complement to the single-strand DNA. As such, nucleic acid molecules can be either double-stranded or single-stranded, and include those nucleic acid molecules that form stable hybrids under "stringent" hybridization conditions with a sequence of the disclosure, and/or with a complement of a sequence of the disclosure. Methods to deduce a complementary sequence are known to those skilled in the art.

The term "polypeptide" includes single-chain polypeptide molecules as well as multiple-polypeptide complexes where individual constituent polypeptides are linked by covalent or non-covalent means. According to the present disclosure, an isolated polypeptide is a polypeptide that has been removed from its natural milieu (i.e., that has been subject to human manipulation) and can include purified proteins, purified peptides, partially purified proteins, partially purified peptides, recombinantly produced proteins or peptides, and synthetically produced proteins or peptides, for example.

As used herein, a recombinant microorganism has a genome which is modified (i.e., mutated or changed) from its normal (i.e., wild-type or naturally occurring) form using recombinant technology. A recombinant microorganism of use in the methods of the present invention can include a microorganism in which nucleic acid molecules have been inserted, deleted, or modified (i.e., mutated, e.g., by insertion, deletion, substitution, and/or inversion of nucleotides), in such a manner that such modification or modifications provide the desired effect within the microorganism. As used herein, genetic modifications which result in a decrease in gene expression, in the function of the gene, or in the function of the gene product (i.e., the protein encoded by the gene) can be referred to as inactivation (complete or partial), deletion, interruption, blockage or down-regulation of a gene. For example, a genetic modification in a gene which results in a decrease in the function of the protein encoded by such gene, can be the result of a complete deletion of the gene (i.e., the gene does not exist in the recombinant microorganism, and therefore the protein does not exist in the recombinant microorganism), a mutation in the gene which results in incomplete or no translation of the protein (e.g., the protein is not expressed), or a mutation in the gene which decreases or abolishes the natural function of the protein (e.g., a protein is expressed which has decreased or no activity (for example, enzymatic activity or action). Genetic modifications which result in an increase in gene expression or function can be referred to as amplification, overproduction, overexpression, activation, enhancement, addition, or up-regulation of a gene.

### Promoters

A promoter is a region of DNA that directs transcription of an associated coding region.

In some embodiments, the promoter is from a microorganism of the phylum Labyrinthulomycota. In some embodiments, the promoter is from a thraustochytrid including, but not limited to: the microorganism deposited as SAM2179 (named "Ulkenia SAM2179" by the depositor), a microorganism of the genus *Ulkenia* or *Thraustochytrium,* or a *Schizochytrium. Schizochytrium* include, but are not limited to, *Schizochytrium aggregatum, Schizochytrium limacinum, Schizochytrium* sp. (S31) (ATCC 20888), *Schizochytrium* sp. (S8) (ATCC 20889), *Schizochytrium* sp. (LC-RM) (ATCC 18915), *Schizochytrium* sp. (SR 21), deposited *Schizochytrium* strain ATCC 28209, and deposited *Schizochytrium* strain IFO 32693.

A promoter can have promoter activity at least in a thraustochytrid, and includes full-length promoter sequences and functional fragments thereof, fusion sequences, and homologues of a naturally occurring promoter. A homologue of a promoter differs from a naturally occurring promoter in that at least one, two, three, or several, nucleotides have been deleted, inserted, inverted, substituted and/or derivatized. A homologue of a promoter can retain activity as a promoter, at least in a thraustochytrid, although the activity can be increased, decreased, or made dependant upon certain stimuli. Promoters can comprise one or more sequence elements that confer developmental and tissue-specific regulatory control or expression.

In some embodiments, an isolated nucleic acid molecule as described herein comprises a PUFA PKS OrfC promoter ("PKS OrfC promoter"; also known as the PFA3 promoter) such as, for example, a polynucleotide sequence represented by SEQ ID NO:3. A PKS OrfC promoter includes a PKS OrfC promoter homologue that is sufficiently similar to a naturally occurring PKS OrfC promoter sequence that the nucleic acid sequence of the homologue is capable of hybridizing under moderate, high, or very high stringency conditions to the complement of the nucleic acid sequence of a naturally occurring PKS OrfC promoter such as, for example, SEQ ID NO:3 or the OrfC promoter of pCL0001 as deposited in ATCC Accession No. PTA-9615.

In some embodiments, an isolated nucleic acid molecule of use in the invention comprises an EF1 short promoter ("EFl short" or "EF1-S" promoter) or EF1 long promoter ("EF1 long" or "EF1-L" promoter) such as, for example, an EF1 short promoter as represented by SEQ ID NO:42, or an EF1 long promoter as represented by SEQ ID NO:43. An EF1 short or EF1 long promoter includes an EF1 short or long promoter homologue that is sufficiently similar to a naturally occurring EF1 short and/or long promoter sequence, respectively, that the nucleic acid sequence of the homologue is capable of hybridizing under moderate, high, or very high stringency conditions to the complement of the nucleic acid sequence of a naturally occurring EF1 short and/or long promoter such as, for example, SEQ ID NO:42 and/or SEQ ID NO:43, respectively, or the EF1 long promoter of pAB0018 as deposited in ATCC Accession No. PTA-9616.

In some embodiments, an isolated nucleic acid molecule of use in the invention comprises a 60S short promoter ("60S short" or "60S-S" promoter) or 60S long promoter ("60S long" or "60S-L" promoter) such as, for example, a 60S short promoter as represented by SEQ ID NO:44, or a 60S long promoter has a polynucleotide sequence represented by SEQ ID NO:45. In some embodiments, a 60S short or 60S long promoter includes a 60S short or 60S long promoter homologue that is sufficiently similar to a naturally occurring 60S short or 60S long promoter sequence, respectively, that the nucleic acid sequence of the homologue is capable of hybridizing under moderate, high, or very high stringency conditions to the complement of the nucleic acid sequence of a naturally occurring 60S short and/or 60S long such as, for example, SEQ ID NO:44 and/or SEQ ID NO:45, respectively, or the 60S long promoter of pAB0011 as deposited in ATCC Accession No. PTA-9614.

In some embodiments, an isolated nucleic acid molecule comprises a Sec1 promoter ("Sec1 promoter") such as, for example, a polynucleotide sequence represented by SEQ ID NO:46. In some embodiments, a Sec1 promoter includes a Sec1 promoter homologue that is sufficiently similar to a naturally occurring Sec1 promoter sequence that the nucleic acid sequence of the homologue is capable of hybridizing under moderate, high, or very high stringency conditions to the complement of the nucleic acid sequence of a naturally occurring Sec1 promoter such as, for example, SEQ ID NO:46,or the Sec1 promoter of pAB0022 as deposited in ATCC Accession No. PTA-9613.

### Terminators

A terminator region is a section of genetic sequence that marks the end of a gene sequence in genomic DNA for transcription.

In some embodiments, the terminator region is from a microorganism of the phylum Labyrinthulomycota. In some embodiments, the terminator region is from a thraustochytrid. In some embodiments, the terminator region is from a *Schizochytrium* or a *Thraustochytrium. Schizochytrium* include, but are not limited to, *Schizochytrium aggregatum, Schizochytrium limacinum, Schizochytrium* sp. (S31) (ATCC 20888), *Schizochytrium* sp. (S8) (ATCC 20889), *Schizochytrium* sp. (LC-RM) (ATCC 18915), *Schizochytrium* sp. (SR 21), deposited strain ATCC 28209, and deposited strain IFO 32693. In some embodiments, the terminator region is a heterologous terminator region, such as, for example, a heterologous SV40 terminator region.

A terminator region can have terminator activity at least in a thraustochytrid and includes full-length terminator sequences and functional fragments thereof, fusion sequences, and homologues of a naturally occurring terminator region. A homologue of a terminator differs from a naturally occurring terminator in that at least one or a few, but not limited to one or a few, nucleotides have been deleted, inserted, inverted, substituted and/or derivatized. In some embodiments, homologues of a terminator retain activity as a terminator region at least in a thraustochytrid, although the activity can be increased, decreased, or made dependent upon certain stimuli.

In some embodiments, an isolated nucleic acid molecule can comprise a terminator region of a PUFA PKS OrfC gene ("PKS OrfC terminator region", also known as the PFA3 terminator) such as, for example, a polynucleotide sequence represented by SEQ ID NO:4. The terminator region disclosed in SEQ ID NO:4 is a naturally occurring (wild-type) terminator sequence from a thraustochytrid microorganism, and, specifically, is a *Schizochytrium* PKS OrfC terminator region and is termed "OrfC terminator element 1." In some embodiments, a PKS OrfC terminator region includes a PKS OrfC terminator region homologue that is sufficiently similar to a naturally occurring PUFA PKS OrfC terminator region that the nucleic acid sequence of a homologue is capable of hybridizing under moderate, high, or very high stringency conditions to the complement of the nucleic acid sequence of a naturally occurring PKS OrfC terminator region such as, for example, SEQ ID NO:4 or the OrfC terminator region of pAB0011 as deposited in ATCC Accession No. PTA-9614.

### Signal Peptides

In some embodiments, an isolated nucleic acid molecule can comprise a polynucleotide sequence encoding a signal peptide of a secreted protein from a microorganism of the phylum Labyrinthulomycota. In some embodiments, the microorganism is a thraustochytrid. In some embodiments, the microorganism is a *Schizochytrium* or a *Thraustochytrium.*

A signal peptide can have secretion signal activity in a thraustochytrid, and includes full-length peptides and functional fragments thereof, fusion peptides, and homologues of a naturally occurring signal peptide. A homologue of a signal peptide differs from a naturally occurring signal peptide in that at least one or a few, but not limited to one or a few, amino acids have been deleted (e.g., a truncated version of the protein, such as a peptide or fragment), inserted, inverted, substituted and/or derivatized (e.g., by glycosylation, phosphorylation, acetylation, myristoylation, prenylation, palmitation, amidation, and/or addition of glycosylphosphatidyl inositol). In some embodiments, homologues of a signal peptide retain activity as a signal at least in a thraustochytrid, although the activity can be increased, decreased, or made dependant upon certain stimuli.

In some embodiments, the isolated nucleic acid molecule comprises a polynucleotide sequence encoding a Na/Pi-IIb2 transporter protein signal peptide. A Na/Pi-IIb2 transporter protein signal peptide can have signal targeting activity at least for a Na/Pi-IIb2 transporter protein at least in a thraustochytrid, and includes full-length peptides and functional fragments thereof, fusion peptides, and homologues of a naturally occurring Na/Pi-IIb2 transporter protein signal peptide. In some embodiments, the Na/Pi-IIb2 transporter protein signal peptide has an amino acid sequence represented by SEQ ID NO:1. In some embodiments, the Na/Pi-IIb2 transporter protein signal peptide has an amino acid sequence represented by SEQ ID NO:15. In some embodiments, the isolated nucleic acid molecule comprises a polynucleotide sequence encoding an isolated amino acid sequence comprising a functional fragment of SEQ ID NO:1 or SEQ ID NO:15 that functions as a signal peptide, at least for a Na/Pi-IIb2 transporter protein, at least in a thraustochytrid. In some embodiments, the isolated nucleic acid molecule comprises SEQ ID NO:2.

Also disclosed herein but not claimed as such is an isolated polypeptide comprising a Na/Pi-IIb2 transporter signal peptide amino acid sequence.

In some embodiments, the isolated nucleic acid molecule comprises a polynucleotide sequence encoding an alpha-1,6-mannosyltransferase (ALG12) signal peptide. An ALG12 signal peptide can have signal targeting activity at least for an ALG12 protein, at least in a thraustochytrid, and includes full-length peptides and functional fragments thereof, fusion peptides, and homologues of a naturally occurring ALG12 signal peptide. In some embodiments, the ALG12 signal peptide has an amino acid sequence represented by SEQ ID NO:59. In some embodiments, the isolated nucleic acid molecule comprises a polynucleotide sequence encoding an isolated amino acid sequence comprising a functional fragment of SEQ ID NO:59 that functions as a signal peptide at least for an ALG12 protein, at least in a thraustochytrid. In some embodiments, the isolated nucleic acid molecule comprises SEQ ID NO:60.

Also disclosed herein but not claimed as such is an isolated polypeptide comprising a ALG12 signal peptide amino acid sequence.

In some embodiments, the isolated nucleic acid molecule comprises a polynucleotide sequence encoding a binding immunoglobulin protein (BiP) signal peptide. A BiP signal peptide can have signal targeting activity at least for a BiP protein, at least in a thraustochytrid, and includes full-length peptides and functional fragments thereof, fusion peptides, and homologues of a naturally occurring BiP signal peptide. In some embodiments, the BiP signal peptide has an amino acid sequence represented by SEQ ID NO:61. In some embodiments, the isolated nucleic acid molecule comprises a polynucleotide sequence encoding an isolated amino acid sequence comprising a functional fragment of SEQ ID NO:61 that functions as a signal peptide at least for a BiP protein, at least in a thraustochytrid. In some embodiments, the isolated nucleic acid molecule comprises SEQ ID NO:62.

Also disclosed herein but not claimed as such is an isolated polypeptide comprising a BiP signal peptide amino acid sequence.

In some embodiments, the isolated nucleic acid molecule comprises a polynucleotide sequence encoding an alpha-1,3-glucosidase (GLS2) signal peptide. A GLS2 signal peptide can have signal targeting activity at least for a GLS2 protein, at least in a thraustochytrid, and includes full-length peptides and functional fragments thereof, fusion peptides, and homologues of a naturally occurring GLS2 signal peptide. In some embodiments, the GLS2 signal peptide has an amino acid sequence represented by SEQ ID NO:63. In some embodiments, the isolated nucleic acid molecule comprises a polynucleotide sequence encoding an isolated amino acid sequence comprising a functional fragment of SEQ ID NO:63 that functions as a signal peptide at least for a GLS2 protein, at least in a thraustochytrid. In some embodiments, the isolated nucleic acid molecule comprises SEQ ID NO:64.

Also disclosed herein but not claimed as such is an isolated polypeptide comprising a GLS2 signal peptide amino acid sequence.

In some embodiments, the isolated nucleic acid molecule comprises a polynucleotide sequence encoding an alpha-1,3-1,6-mannosidase-like signal peptide. A alpha-1,3-1,6-mannosidase-like signal peptide can have signal targeting activity at least for an alpha-1,3-1,6-mannosidase-like protein, at least in a thraustochytrid, and includes full-length peptides and functional fragments thereof, fusion peptides, and homologues of a naturally occurring alpha-1,3-1,6-mannosidase-like signal peptide. In some embodiments, the alpha-1,3-1,6-mannosidase-like signal peptide has an amino acid sequence represented by SEQ ID NO:65. In some embodiments, the isolated nucleic acid molecule comprises a polynucleotide sequence encoding an isolated amino acid sequence comprising a functional fragment of SEQ ID NO:65 that functions as a signal peptide at least for an alpha-1,3-1,6-mannosidase-like protein, at least in a thraustochytrid. In some embodiments, the isolated nucleic acid molecule comprises SEQ ID NO:66.

Also disclosed herein but not claimed as such is an isolated polypeptide comprising a alpha-1,3-1,6-mannosidase-like signal peptide amino acid sequence.

In some embodiments, the isolated nucleic acid molecule comprises a polynucleotide sequence encoding an alpha-1,3-1,6-mannosidase-like #1 signal peptide. An alpha-1,3-1,6-mannosidase-like #1 signal peptide can have signal targeting activity at least for an alpha-1,3-1,6-mannosidase-like #1 protein, at least in a thraustochytrid, and includes full-length peptides and functional fragments thereof, fusion peptides, and homologues of a naturally occurring alpha-1,3-1,6-mannosidase-like #1 signal peptide. In some embodiments, the alpha-1,3-1,6-mannosidase-like #1 signal peptide has an amino acid sequence represented by SEQ ID NO:67. In some embodiments, the isolated nucleic acid molecule comprises a polynucleotide sequence encoding an isolated amino acid sequence comprising a functional fragment of SEQ ID NO:67 that functions as a signal peptide at least for an alpha-1,3-1,6-mannosidase-like #1 protein, at least in a thraustochytrid. In some embodiments, the isolated nucleic acid molecule comprises SEQ ID NO:68.

Also disclosed herein but not claimed as such is an isolated polypeptide comprising a alpha-1,3-1,6-mannosidase-like #1 signal peptide amino acid sequence.

In some embodiments, the isolated nucleic acid molecule comprises a polynucleotide sequence encoding an alpha-1,2-mannosidase-like signal peptide. An alpha-1,2-mannosidase-like signal peptide can have signal targeting activity at least for an alpha-1,2-mannosidase-like protein, at least in a thraustochytrid, and includes full-length peptides and functional fragments thereof, fusion peptides, and homologues of a naturally occurring alpha-1,2-mannosidase-like signal peptide. In some embodiments, the alpha-1,2-mannosidase-like signal peptide has an amino acid sequence represented by SEQ ID NO:69. In some embodiments, the isolated nucleic acid molecule comprises a polynucleotide sequence encoding an isolated amino acid sequence comprising a functional fragment of SEQ ID NO:69 that functions as a signal peptide at least for an alpha-1,2-mannosidase-like protein, at least in a thraustochytrid. In some embodiments, the isolated nucleic acid molecule comprises SEQ ID NO:70.

Also disclosed herein but not claimed as such is an isolated polypeptide comprising a alpha-1,2-mannosidase-like signal peptide amino acid sequence.

In some embodiments, the isolated nucleic acid molecule comprises a polynucleotide sequence encoding a beta-xylosidase-like signal peptide. A beta-xylosidase-like signal peptide can have signal targeting activity at least for a beta-xylosidase-like protein, at least in a thraustochytrid, and includes full-length peptides and functional fragments thereof, fusion peptides, and homologues of a naturally occurring beta-xylosidase-like signal peptide. In some embodiments, the beta-xylosidase-like signal peptide has an amino acid sequence represented by SEQ ID NO:71. In some embodiments, the isolated nucleic acid molecule comprises a polynucleotide sequence encoding an isolated amino acid sequence comprising a functional fragment of SEQ ID NO:71 that functions as a signal peptide at least for a beta xylosidase-like protein, at least in a thraustochytrid. In some embodiments, the isolated nucleic acid molecule comprises SEQ ID NO:72.

Also disclosed herein but not claimed as such is an isolated polypeptide comprising a beta-xylosidase-like signal peptide amino acid sequence.

In some embodiments, the isolated nucleic acid molecule comprises a polynucleotide sequence encoding a carotene synthase signal peptide. A carotene synthase signal peptide can have signal targeting activity at least for a carotene synthase protein, at least in a thraustochytrid, and includes full-length peptides and functional fragments thereof, fusion peptides, and homologues of a naturally occurring carotene synthase signal peptide. In some embodiments, the carotene synthase signal peptide has an amino acid sequence represented by SEQ ID NO:73. In some embodiments, the isolated nucleic acid molecule comprises a polynucleotide sequence encoding an isolated amino acid sequence comprising a functional fragment of SEQ ID NO:73 that functions as a signal peptide at least for a carotene synthase protein, at least in a thraustochytrid. In some embodiments, the isolated nucleic acid molecule comprises SEQ ID NO:74.

Also disclosed herein but not claimed as such is an isolated polypeptide comprising a carotene synthase signal peptide amino acid sequence.

In some embodiments, the isolated nucleic acid molecule comprises a polynucleotide sequence encoding a Sec1 protein ("Sec1") signal peptide. A Sec1 signal peptide can have secretion signal activity at least for a Sec1 protein at least in a thraustochytrid, and includes full-length peptides and functional fragments thereof, fusion peptides, and homologues of a naturally occurring Sec1 signal peptide. In some embodiments, the Sec1 signal peptide is represented by SEQ ID NO:37. In some embodiments, the isolated nucleic acid molecule comprises a polynucleotide sequence encoding an isolated amino acid sequence comprising a functional fragment of SEQ ID NO:37 that functions as a signal peptide, at least for a Sec1 protein, at least in a thraustochytrid. In some embodiments, the isolated nucleic acid molecule comprises SEQ ID NO:38.

Also disclosed herein but not claimed as such is an isolated polypeptide comprising a Sec1 signal peptide amino acid sequence.

In some embodiments, an isolated nucleic acid molecule can comprise a promoter sequence, a terminator sequence, and/or a signal peptide sequence that is at least 90%, 95%, 96%, 97%, 98%, or 99% identical to any of the promoter, terminator, and/or signal peptide sequences described herein.

In some embodiments, an isolated nucleic acid molecule comprises an OrfC promoter, EF1 short promoter, EF1 long promoter, 60S short promoter, 60S long promoter, Sec1 promoter, PKS OrfC terminator region, sequence encoding a Na/Pi-IIb2 transporter protein signal peptide, or sequence encoding a Sec1 transport protein signal peptide that is operably linked to the 5' end of a nucleic acid sequence encoding a heterologous polypeptide. Recombinant vectors (including, but not limited to, expression vectors), expression cassettes, and host cells can also comprise an OrfC promoter, EF1 short promoter, EF1 long promoter, 60S short promoter, 60S long promoter, Sec1 promoter, PKS OrfC terminator region, sequence encoding a Na/Pi-IIb2 transporter protein signal peptide, or sequence encoding a Sec1 transport protein signal peptide that is operably linked to the 5' end of a nucleic acid sequence encoding a heterologous polypeptide.

As used herein, unless otherwise specified, reference to a percent (%) identity (and % identical) refers to an evaluation of homology which is performed using: (1) a BLAST 2.0 Basic BLAST homology search using blastp for amino acid searches and blastn for nucleic acid searches with standard default parameters, wherein the query sequence is filtered for low complexity regions by default (*see,* for example, Altschul, S., et al., Nucleic Acids Res. 25:3389-3402 (1997));
(2) a BLAST 2 alignment using the parameters described below; (3) and/or PSI-BLAST (Position-Specific Iterated BLAST) with the standard default parameters. It is noted that due to some differences in the standard parameters between BLAST 2.0 Basic BLAST and BLAST 2, two specific sequences might be recognized as having significant homology using the BLAST 2 program, whereas a search performed in BLAST 2.0 Basic BLAST using one of the sequences as the query sequence may not identify the second sequence in the top matches. In addition, PSI-BLAST provides an automated, easy-to-use version of a "profile" search, which is a sensitive way to look for sequence homologues. The program first performs a gapped BLAST database search. The PSI-BLAST program uses the information from any significant alignments returned to construct a position-specific score matrix, which replaces the query sequence for the next round of database searching. Therefore, it is to be understood that percent identity can be determined by using any one of these programs.

Two specific sequences can be aligned to one another using BLAST 2 sequence as described, for example, in Tatusova and Madden, FEMS Microbiol. Lett. 174:247-250 (1999). BLAST 2 sequence alignment is performed in blastp or blastn using the BLAST 2.0 algorithm to perform a Gapped BLAST search (BLAST 2.0) between the two sequences allowing for the introduction of gaps (deletions and insertions) in the resulting alignment. BLAST 2 sequence alignment may be performed using the standard default parameters as follows.

For blastn, using 0 BLOSUM62 matrix:
Reward for match = 1
Penalty for mismatch = -2
Open gap (5) and extension gap (2) penalties gap x_dropoff(50) expect (10) word size (11) filter (on).
For blastp, using 0 BLOSUM62 matrix:
   Open gap (11) and extension gap (1) penalties
   gap x_dropoff(50) expect (10) word size (3) filter (on).

As used herein, hybridization conditions refer to standard hybridization conditions under which nucleic acid molecules are used to identify similar nucleic acid molecules. See, for example, Sambrook J. and Russell D. (2001) Molecular cloning: A laboratory manual, 3rd ed. Cold Spring Harbor Laboratory Press, Cold Spring Harbor, New York.

In addition, formulae to calculate the appropriate hybridization and wash conditions to achieve hybridization permitting varying degrees of mismatch of nucleotides are disclosed, for example, in Meinkoth et al., Anal. Biochem. 138:261-284 (1984).

One of skill in the art can use the formulae in Meinkoth *et al.,* for example, to calculate the appropriate hybridization and wash conditions to achieve particular levels of nucleotide mismatch. Such conditions will vary, depending on whether DNA:RNA or DNA:DNA hybrids are being formed. Calculated melting temperatures for DNA:DNA hybrids are 10°C less than for DNA:RNA hybrids. In particular instances, stringent hybridization conditions for DNA:DNA hybrids include hybridization at an ionic strength of 6X SSC (0.9 M Na⁺) at a temperature of between 20°C and 35°C (lower stringency), between 28°C and 40°C (more stringent), and between 35°C and 45°C (even more stringent), with appropriate wash conditions. In particular instances, stringent hybridization conditions for DNA:RNA hybrids include hybridization at an ionic strength of 6X SSC (0.9 M Na⁺) at a temperature of between 30°C and 45°C, between 38°C and 50°C, and between 45°C and 55°C, with similarly stringent wash conditions. These values are based on calculations of a melting temperature for molecules larger than about 100 nucleotides, 0% formamide, and a G+C content of about 40%. Alternatively, Tₘ can be calculated empirically as set forth in Sambrook *et al.* In general, the wash conditions should be as stringent as possible, and should be appropriate for the chosen hybridization conditions. For example, hybridization conditions can include a combination of salt and temperature conditions that are approximately 20-25°C below the calculated Tₘ of a particular hybrid, and wash conditions typically include a combination of salt and temperature conditions that are approximately 12-20°C below the calculated Tₘ of the particular hybrid. One example of hybridization conditions suitable for use with DNA:DNA hybrids includes a 2-24 hour hybridization in 6X SSC (50% formamide) at 42°C, followed by washing steps that include one or more washes at room temperature in 2X SSC, followed by additional washes at higher temperatures and lower ionic strength (e.g., at least one wash as 37°C in 0.1X - 0.5X SSC, followed by at least one wash at 68°C in 0.1X - 0.5X SSC).

### Heterologous Polypeptides

The term "heterologous" as used herein refers to a sequence that is not naturally found in the microalgal host cell. Heterologous polypeptides that may be produced by a recombinant host cell of the disclosure include, but are not limited to, therapeutic proteins. A "therapeutic protein" as used herein includes proteins that are useful for the treatment or prevention of diseases, conditions, or disorders in animals and humans. In the methods of the invention, the heterologous polypeptide is a viral NA protein comprising a membrane domain.

Therapeutic proteins include, but are not limited to, biologically active proteins, e.g., enzymes, antibodies, or antigenic proteins. .

Influenza is the leading cause of death in humans due to a respiratory virus. Common symptoms include fever, sore throat, shortness of breath, and muscle soreness, among others. Influenza viruses are enveloped viruses that bud from the plasma membrane of infected mammalian and avian cells. They are classified into types A, B, or C, based on the nucleoproteins and matrix protein antigens present. Influenza type A viruses can be further divided into subtypes according to the combination of HA and NA surface glycoproteins presented. HA is an antigenic glycoprotein, and plays a role in binding the virus to cells that are being infected. NA removes terminal sialic acid residues from glycan chains on host cell and viral surface proteins, which prevents viral aggregation and facilitates virus mobility.

The influenza viral HA protein is a homo trimer with a receptor binding pocket on the globular head of each monomer, and the influenza viral NA protein is a tetramer with an enzyme active site on the head of each monomer. Currently, 16 HA (Hl-H16) and 9 NA (N1-N9) subtypes are recognized. Each type A influenza virus presents one type of HA and one type of NA glycoprotein. Generally, each subtype exhibits species specificity; for example, all HA and NA subtypes are known to infect birds, while only subtypes H1 , H2, H3, H5, H7, H9, H10, N1 , N2, N3 and N7 have been shown to infect humans. Influenza viruses are characterized by the type of HA and NA that they carry, *e.g.,* H1N1, H5N1, H1N2, H1N3, H2N2, H3N2, H4N6, H5N2, H5N3, H5N8, H6N1, H7N7, H8N4, H9N2, H10N3, H11N2, H11N9, H12N5, H13N8, H15N8, H16N3, etc. Subtypes are further divided into strains; each genetically distinct virus isolate is usually considered to be a separate strain, e.g., influenza A/Puerto Rico/8/34/Mount Sinai(H1N1) and influenza A/Vietnam/1203/2004(H5N1).

An influenza virus HA protein is translated in cells as a single protein, which after cleavage of the signal peptide is an approximately 62 kDa protein (by conceptual translation) referred to as HA0 (i.e., hemagglutinin precursor protein). For viral activation, hemagglutinin precursor protein (HA0) must be cleaved by a trypsin-like serine endoprotease at a specific site, normally coded for by a single basic amino acid (usually arginine) between the HA1 and HA2 polypeptides of the protein. In the specific example of the A/Puerto Rico/8/34 strain, this cleavage occurs between the arginine at amino acid 343 and the glycine at amino acid 344. After cleavage, the two disulfide-bonded protein polypeptides produce the mature form of the protein subunits as a prerequisite for the conformational change necessary for fusion and hence viral infectivity.

Expression
of the HA protein in a microalgal host cell such as *Schizochytrium,* can result in proper cleavage of the HA0 protein into functional HA1 and HA2 polypeptides without addition of an exogenous protease. Such cleavage of hemagglutinin in a non-vertebrate expression system without addition of exogenous protease has not been previously demonstrated. However, the production of HA protein is not claimed as such.

A viral F protein can comprise a single-pass transmembrane domain near the C-terminus. The F protein can be split into two peptides at the Furin cleavage site (amino acid 109). The first portion of the protein designated F2 contains the N-terminal portion of the complete F protein. The remainder of the viral F protein containing the C-terminal portion of the F protein is designated F1. The F1 and/or F2 regions can be fused individually to heterologous sequences, such as, for example, a sequence encoding a heterologous signal peptide. Vectors containing the F1 and F2 portions of the viral F protein can be expressed individually or in combination. A vector expressing the complete F protein can be co-expressed with the furin enzyme that will cleave the protein at the furin cleavage site. Alternatively, the sequence encoding the furin cleavage site of the F protein can be replaced with a sequence encoding an alternate protease cleavage site that is recognized and cleaved by a different protease. The F protein containing an alternate protease cleavage site can be co-expressed with a corresponding protease that recognizes and cleaves the alternate protease cleavage site. Production of F protein is not claimed as such.

An HA, NA, F, G, E, gp120, gp41, or matrix protein may be a full-length protein, a fragment, a variant, a derivative, or an analogue thereof.

An HA, NA, F, G, E, gp120, gp41, or matrix protein may be a polypeptide comprising an amino acid sequence or a polynucleotide encoding a polypeptide comprising an amino acid sequence at least 90%, at least 91%, at least 92%, at least 93%, at least 94%, at least 95%, at least 96%, at least 97%, at least 98%, at least 99%, or 100% identical to a known sequence for the respective viral proteins, wherein the polypeptide is recognizable by an antibody that specifically binds to the known sequence. The HA sequence, for example, can be a full-length HA protein which consists essentially of the extracellular (ECD) domain, the transmembrane (TM) domain, and the cytoplasmic (CYT) domain; or a fragment of the entire HA protein which consists essentially of the HA1 polypeptide and the HA2 polypeptide, e.g., produced by cleavage of a full-length HA; or a fragment of the entire HA protein which consists essentially of the HA1 polypeptide, HA2 polypeptide and the TM domain; or a fragment of the entire HA protein which consists essentially of the CYT domain; or a fragment of the entire HA protein which consists essentially of the TM domain; or a fragment of the entire HA protein which consists essentially of the HA1 polypeptide; or a fragment of the entire HA protein which consists essentially of the HA2 polypeptide. The HA sequence can also include an HA1/HA2 cleavage site. The HA1/HA2 cleavage site can be located between the HA1 and HA2 polypeptides, but also can be arranged in any order relative to the other sequences of the polynucleotide or polypeptide construct. The viral proteins can be from a pathogenic virus strain. Production of HA, F, G, E, gp120, gp41 and matrix protein is not claimed as such.

In some embodiments, a heterologous polypeptide of the invention is a fusion polypeptide comprising a full-length NA protein, or a fragment, variant, derivative, or analogue thereof.

Hemagglutination activity can be determined by measuring agglutination of red blood cells. Hemagglutination and subsequent precipitation of red blood cells results from hemagglutinins being adsorbed onto the surface of red blood cells. Clusters of red blood cells, distinguishable to the naked eye as heaps, lumps, and/or clumps, are formed during hemagglutination. Hemagglutination is caused by the interaction of the agglutinogens present in red blood cells with plasma that contains agglutinins. Each agglutinogen has a corresponding agglutinin. A hemagglutination reaction is used, *e.g.,* to determine antiserum activity or type of virus. A distinction is made between active hemagglutination, which is caused by the direct action of an agent on the red blood cells, and passive hemagglutination, caused by a specific antiserum to the antigen previously adsorbed by the red blood cells. The amount of hemagglutination activity in a sample can be measured, e.g., in hemagglutination activity units (HAU). Hemagglutination may be caused by, e.g., the polysaccharides of the causative bacteria of tuberculosis, plague, and tularemia, by the polysaccharides of the colon bacillus, and by the viruses of influenza, mumps, pneumonia of white mice, swine and horse influenza, smallpox vaccine, yellow fever, and other hemagglutination-inducing diseases.

### Microalgal Extracellular Bodies

Method of producing and using microalgal extracellular bodies are not claimed as such.

Microalgal cells may produce a microalgal extracellular body, wherein the extracellular body is discontinuous with the plasma membrane. By "discontinuous with the plasma membrane" is meant that the microalgal extracellular body is not connected to the plasma membrane of a host cell. In some instances, the extracellular body is a membrane. In some instances, the extracellular body is a vesicle, micelle, membrane fragment, membrane aggregate, or a mixture thereof. The term "vesicle" as used herein refers to a closed structure comprising a lipid bilayer (unit membrane), e.g., a bubble-like structure formed by a cell membrane. The term "membrane aggregate" as used herein refers to any collection of membrane structures that become associated as a single mass. A membrane aggregate can be a collection of a single type of membrane structure such as, but not limited to, a collection of membrane vesicles, or can be a collection of more than a single type of membrane structure such as, but not limited to, a collection of at least two of a vesicle, micelle, or membrane fragment. The term "membrane fragment" as used herein refers to any portion of a membrane capable of comprising a heterologous polypeptide as described herein. In some instances, a membrane fragment is a membrane sheet. In some instances,
the extracellular body is a mixture of a vesicle and a membrane fragment. In some instances, the extracellular body is a vesicle. In some instances, the vesicle is a collapsed vesicle. In some instances, the vesicle is a virus-like particle. In some instances, the extracellular body is an aggregate of biological materials comprising native and heterologous polypeptides produced by the host cell. In some instances,
the extracellular body is an aggregate of native and heterologous polypeptides. In some instances, the extracellular body is an aggregate of heterologous polypeptides.

In some instances, the ectoplasmic net of a microalgal host cell becomes fragmented during culturing of a microalgal host cell, resulting in the formation of a microalgal extracellular body. In some instances, the microalgal extracellular body is formed by fragmentation of the ectoplasmic net of a microalgal host cell as a result of hydrodynamic forces in the stirred media that physically shear ectoplasmic net membrane extensions.

In some instances, the microalgal extracellular body is formed by extrusion of a microalgal membrane, such as, but not limited to, extrusion of a plasma membrane, an ectoplasmic net, a pseudorhizoid, or a combination thereof, wherein the extruding membrane becomes separated from the plasma membrane.

In some instances, the microalgal extracellular bodies are vesicles or micelles having different diameters, membrane fragments having different lengths, or a combination thereof.

In some instances, the extracellular body is a vesicle having a diameter from 10 nm to 2500 nm, 10 nm to 2000 nm, 10 nm to 1500 nm, 10 nm to 1000 nm, 10 nm to 500 nm, 10 nm to 300 nm, 10 nm to 200 nm, 10 nm to 100 nm, 10 nm to 50 nm, 20 nm to 2500 nm, 20 nm to 2000 nm, 20 nm to 1500 nm, 20 nm to 1000 nm, 20 nm to 500 nm, 20 nm to 300 nm, 20 nm to 200 nm, 20 nm to 100 nm, 50 nm to 2500 nm, 50 nm to 2000 nm, 50 nm to 1500 nm, 50 nm to 1000 nm, 50 nm to 500 nm, 50 nm to 300 nm, 50 nm to 200 nm, 50 nm to 100 nm, 100 nm to 2500 nm, 100 nm to 2000 nm, 100 nm to 1500 nm, 100 nm to 1000 nm, 100 nm to 500 nm, 100 nm to 300 nm, 100 nm to 200 nm, 500 nm to 2500 nm, 500 nm to 2000 nm, 500 nm to 1500 nm, 500 nm to 1000 nm, 2000 nm or less, 1500 nm or less, 1000 nm or less, 500 nm or less, 400 nm or less, 300 nm or less, 200 nm or less, 100 nm or less, or 50 nm or less.

Non-limiting fermentation conditions for producing microalgal extracellular bodies from thraustochytrid host cells are shown below in **Table 1**:

**Table 1: Vessel Media**

| Ingredient | Concentration | | Ranges |
|---|---|---|---|
| Na₂SO₄ | g/L | 13.62 | 0-50, 15-45, or 25-35 |
| K2SO4 | g/L | 0.72 | 0-25, 0.1-10, or 0.5-5 |
| KCl | g/L | 0.56 | 0-5, 0.25-3, or 0.5-2 |
| MgSO₄·7H₂O | g/L | 2.27 | 0-10, 1-8, or 2-6 |
| (NH₄)₂SO₄ | g/L | 17.5 | 0-50, 0.25-30, or 5-20 |
| CaCl₂·2H₂O | g/L | 0.19 | 0.1-5, 0.1-3, or 0.15-1 |
| KH₂PO₄ | g/L | 6.0 | 0-20, 0.1-10, or 1-7 |

| Post autoclave (Metals) | | | |
|---|---|---|---|
| Citric acid | mg/L | 3.50 | 0.1-5000, 1-3000, or 3-2500 |
| FeSO₄·7H₂O | mg/L | 51.5 | 0.1-1000, 1-500, or 5-100 |
| MnCl₂·4H₂O | mg/L | 3.10 | 0.1-100, 1-50, or 2-25 |
| ZnSO₄·7H₂O | mg/L | 6.20 | 0.1-100, 1-50, or 2-25 |
| CoCl₂·6H₂O | mg/L | 0.04 | 0-1, 0.001-0.1, or 0.01-0.1 |
| Na₂MoO₄·2H₂O | mg/L | 0.04 | 0.001-1, 0.005-0.5, or 0.01-0.1 |
| CuSO₄·5H₂O | mg/L | 2.07 | 0.1-100, 0.5-50, or 1-25 |
| NiSO₄·6H₂O | mg/L | 2.07 | 0.1-100, 0.5-50, or 1-25 |

| Post autoclave (Vitamins) | | | |
|---|---|---|---|
| Thiamine** | mg/L | 9.75 | 0.1-100, 1-50, or 5-25 |
| Vitamin B12** | mg/L | 0.16 | 0.01-100, 0.05-5, or 0.1-1.0 |
| Ca½-pantothenate** | mg/L | 3.33 | 0.1-100, 0.1-50, or 1-10 |
| ** filter sterilized and added post-autoclave | | | |

| Post autoclave (Carbon) | | | |
|---|---|---|---|
| Glucose | g/L | 20.0 | 5-150, 10-100, or 20-50 |
| Nitrogen Feed: | | | |

| Ingredient | Concentration | | |
|---|---|---|---|
| NH₄OH | mL/L | 23.6 | 5-150, 10-100, 15-50 |

General cultivation conditions for producing microalgal extracellular bodies include the following:

| | |
|---|---|
| pH ; | 5.5-9.5, 6.5-8.0, or 6.3-7.3 |
| temperature: | 15 °C-45 °C, 18 °C-35 °C, or 20 °C-30 °C |
| dissolved oxygen: | 0.1%-100% saturation, 5%-50% saturation, or 10%-30% saturation |
| glucose controlled: | 5 g/L-100 g/L, 10 g/L-40 g/L, or 15 g/L-35 g/L. |

In some instances, the microalgal extracellular body is produced from a Labyrinthulomycota host cell. In some instances, the microalgal extracellular body is produced from a Labyrinthulae host cell. In some instances, the microalgal extracellular body is produced from a thraustochytrid host cell. In some instances, the microalgal extracellular body is produced from a *Schizochytrium* or *Thraustochytrium.*

A microalgal extracellular body may comprise a heterologous polypeptide, wherein the extracellular body is discontinuous with a plasma membrane of a microalgal host cell.

In some instances, a microalgal extracellular body comprises a polypeptide that is also associated with a plasma membrane of a microalgal host cell. In some instances, a polypeptide associated with a plasma membrane of a microalgal host cell includes a native membrane polypeptide, a heterologous polypeptide, and a combination thereof.

In some instances, the heterologous polypeptide is contained within a microalgal extracellular body. In the methods of the present invention, a viral NA protein is secreted.

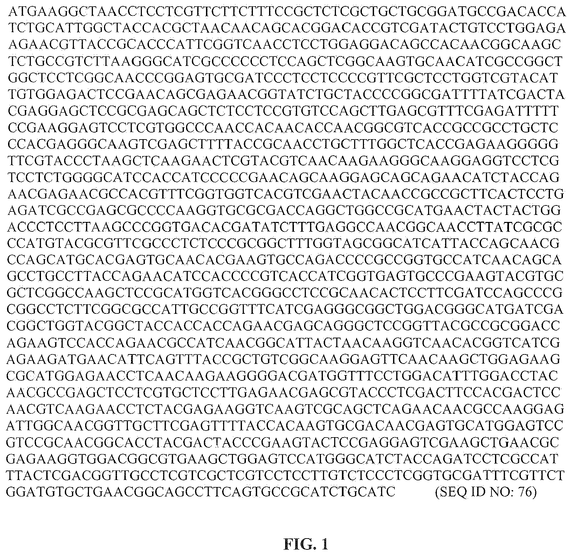

In the present invention, the viral NA protein comprises a membrane domain. The term "membrane domain" as used herein refers to any domain within a polypeptide that targets the polypeptide to a membrane and/or allows the polypeptide to maintain association with a membrane and includes, but is not limited to, a transmembrane domain (e.g., a single or multiple membrane spanning region), an integral monotopic domain, a signal anchor sequence, an ER signal sequence, an N-terminal or internal or C-terminal stop transfer signal, a glycosylphosphatidylinositol anchor, and combinations thereof. A membrane domain can be located at any position in the polypeptide, including the N-terminal, C-terminal, or middle of the polypeptide. A membrane domain can be associated with permanent or temporary attachment of a polypeptide to a membrane. In some embodiments, a membrane domain can be cleaved from a membrane protein. In some embodiments, the membrane domain is a signal anchor sequence. In some embodiments, the membrane domain is any of the signal anchor sequences shown in **FIG.** 1**3**, or an anchor sequence derived therefrom. In some embodiments, the membrane domain is a viral signal anchor sequence.

In some embodiments, the heterologous polypeptide is a polypeptide that naturally comprises a membrane domain. In some embodiments, the heterologous polypeptide does not naturally comprise a membrane domain but has been recombinantly fused to a membrane domain. In some embodiments, the heterologous polypeptide is an otherwise soluble protein that has been fused to a membrane domain.

In some embodiments, the membrane domain is a microalgal membrane domain. In some embodiments, the membrane domain is a Labyrinthulomycota membrane domain. In some embodiments, the membrane domain is a thraustochytrid membrane domain. In some embodiments, the membrane domain is a *Schizochytrium* or *Thraustochytrium* membrane domain. In some embodiments, the membrane domain comprises a signal anchor sequence from *Schizochytrium* alpha-1,3-mannosyl-beta-1,2-GlcNac-transferase-I-like protein #1 (SEQ ID NO:78), *Schizochytrium* beta-1,2-xylosyltransferase-like protein #1 (SEQ ID NO:80), *Schizochytrium* beta-1,4-xylosidase-like protein (SEQ ID NO:82), or *Schizochytrium* galactosyltransferase-like protein #5 (SEQ ID NO:84).

In the present invention, the heterologous polypeptide is a membrane protein. The term "membrane protein" as used herein refers to any protein associated with or bound to a cellular membrane. As described by Chou and Elrod, Proteins: Structure, Function and Genetics 34:137-153 (1999), for example, membrane proteins can be classified into various general types:
1) Type 1 membrane proteins: These proteins have a single transmembrane domain in the mature protein. The N-terminus is extracellular, and the C-terminus is cytoplasmic. The N-terminal end of the proteins characteristically has a classic signal peptide sequence that directs the protein for import to the ER. The proteins are subdivided into Type Ia (containing a cleavable signal sequence) and Type Ib (without a cleavable signal sequence). Examples of Type I membrane proteins include, but are not limited to: Influenza HA, insulin receptor, glycophorin, LDL receptor, and viral G proteins.
2) Type II membrane proteins: For these single membrane domain proteins, the C-terminus is extracellular, and the N-terminus is cytoplasmic. The N-terminus can have a signal anchor sequence. Examples of this protein type include, but are not limited to: Influenza Neuraminidase, Golgi galactosyltransferase, Golgi sialyltransferase, Sucrase-isomaltase precursor, Asialoglycoprotein receptor, and Transferrin receptor.
3) Multipass transmembrane proteins: In Type I and II membrane proteins the polypeptide crosses the lipid bilayer once, whereas in multipass membrane proteins the polypeptide crosses the membrane multiple times. Multipass transmembrane proteins are also subdivided into Types IIIa and IIIb. Type IIIa proteins have cleavable signal sequences. Type IIIb proteins have their amino termini exposed on the exterior surface of the membrane, but do not have a cleavable signal sequence. Type IIIa proteins include, but are not limited to, the M and L peptides of the photoreaction center. Type IIIb proteins include, but are not limited to, cytochrome P450 and leader peptidase of *E. coli.* Additional examples of multipass transmembrane proteins are membrane transporters, such as sugar transporters (glucose, xylose), and ion transporters.
4) Lipid chain anchored membrane proteins: These proteins are associated with the membrane bilayer by means of one or more covalently attached fatty acid chains or other types of lipid chains called prenyl groups.
5) GPI-anchored membrane proteins: These proteins are bound to the membrane by a glycosylphosphatidylinositol (GPI) anchor.
6) Peripheral membrane proteins: These proteins are bound to the membrane indirectly by noncovalent interactions with other membrane proteins.

In some embodiments, the membrane domain is the membrane domain of a HA protein.

In some embodiments, the heterologous polypeptide (viral NA protein comprising a membrane domain) comprises a native signal
anchor sequence or a native membrane domain from a wild-type polypeptide corresponding to the heterologous polypeptide. In some embodiments, the heterologous polypeptide is fused to a heterologous signal anchor sequence or a heterologous membrane domain that is different from the native signal anchor sequence or native membrane domain. In some embodiments, the heterologous polypeptide comprises a heterologous signal anchor sequence or a heterologous membrane domain, while a wild-type polypeptide corresponding to the heterologous polypeptide does not comprise any signal anchor sequence or membrane domain. In some embodiments, the heterologous polypeptide comprises a *Schizochytrium* signal anchor sequence. In some embodiments, the heterologous polypeptide comprises a HA membrane domain. In some embodiments, the heterologous polypeptide is a therapeutic polypeptide.

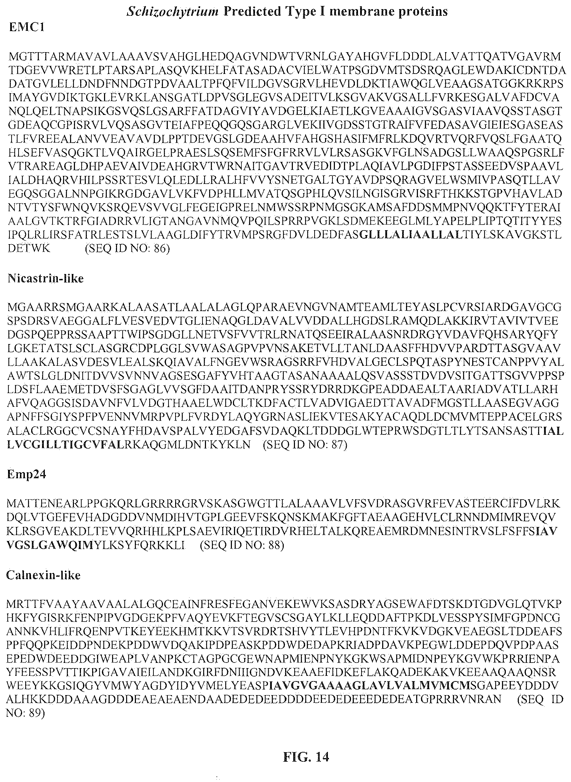

In some embodiments, the membrane domain is a membrane domain from any of the Type I membrane proteins shown in **FIG. 14**, or a membrane domain derived therefrom. In some embodiments, a heterologous polypeptide is a fusion polypeptide comprising the membrane spanning region in the C-terminus of any of the membrane proteins shown in **FIG. 14****.** In some embodiments, the C-terminus side of the membrane spanning region is further modified by replacement with a similar region from a viral protein.

In some embodiments, the heterologous polypeptide is a glycoprotein. In some embodiments, the heterologous polypeptide has a glycosylation pattern characteristic of expression in a Labyrinthulomycota cell. In some embodiments, the heterologous polypeptide has a glycosylation pattern characteristic of expression in a thraustochytrid cell. In some embodiments, a heterologous polypeptide expressed in the microalgal host cell is a glycoprotein having a glycosylation pattern that more closely resembles mammalian glycosylation patterns than proteins produced in yeast or *E. coli.* In some embodiments, the glycosylation pattern comprises a N-linked glycosylation pattern. In some embodiments, the glycoprotein comprises high-mannose oligosaccharides. In some embodiments, the glycoprotein is substantially free of sialic acid. The term "substantially free of sialic acid" as used herein means less than 10%, less than 9%, less than 8%, less than 7%, less than 6%, less than 5%, less than 4%, less than 3%, less than 2%, or less than 1% of sialic acid. In some embodiments, sialic acid is absent from the glycoprotein.

In some instances, a microalgal extracellular body of the disclosure comprising a heterologous polypeptide is produced at commercial or industrial scale.

The present disclosure is also directed to a composition comprising any of the microalgal extracellular bodies of the disclosure as described herein and an aqueous liquid carrier.

In some instances, a microalgal extracellular body of the disclosure comprising a heterologous polypeptide is recovered from the culture medium or fermentation medium in which the microalgal host cell is grown. In some instances,
a microalgal extracellular body of the disclosure can be isolated in "substantially pure" form. As used herein, "substantially pure" refers to a purity that allows for the effective use of the microalgal extracellular body as a commercial or industrial product.

A method of producing a microalgal extracellular body comprising a heterologous polypeptide may comprise:
(a) expressing a heterologous polypeptide in a microalgal host cell, wherein the heterologous polypeptide comprises a membrane domain, and (b) culturing the host cell under culture conditions sufficient to produce a microalgal extracellular body comprising the heterologous polypeptide, wherein the extracellular body is discontinuous with a plasma membrane of the host cell.

A method of producing a composition comprising a microalgal extracellular body and a heterologous polypeptide may comprise: (a) expressing a heterologous polypeptide in a microalgal host cell, wherein the heterologous polypeptide comprises a membrane domain, and (b) culturing the host cell under culture conditions sufficient to produce a microalgal extracellular body comprising the heterologous polypeptide, wherein the extracellular body is discontinuous with a plasma membrane of the host cell, wherein the composition is produced as the culture supernatant comprising the extracellular body. In some instances, the method further comprises removing the culture supernatant and resuspending the extracellular body in an aqueous liquid carrier. In some instances, the composition is used as a vaccine.

### Microalgal Extracellular Bodies Comprising Viral Polypeptides

Virus envelope proteins are membrane proteins that form the outer layer of virus particles. The synthesis of these proteins utilizes membrane domains, such as cellular targeting signals, to direct the proteins to the plasma membrane. Envelope coat proteins fall into several major groups, which include but are not limited to: H or HA (hemagglutinin) proteins, N or NA (neuraminidase) proteins, F (fusion) proteins, G (glycoprotein) proteins, E or env (envelope) protein, gp120 (glycoprotein of 120 kDa), and gp41 (glycoprotein of 41 kDa). Structural proteins commonly referred to as "matrix" proteins serve to help stabilize the virus. Matrix proteins include, but are not limited to, M1, M2 (a membrane channel protein), and Gag. Both the envelope and matrix proteins can participate in the assembly and function of the virus. For example, the expression of virus envelope coat proteins alone or in conjunction with viral matrix proteins can result in the formation of virus-like particles (VLPs).

Viral vaccines are often made from inactivated or attenuated preparations of viral cultures corresponding to the disease they are intended to prevent, and generally retain viral material such as viral genetic material. Generally, a virus is cultured from the same or similar cell type as the virus might infect in the wild. Such cell culture is expensive and often difficult to scale. To address this problem, certain specific viral protein antigens are instead expressed by a transgenic host, which can be less costly to culture and more amenable to scale. However, viral proteins are typically integral membrane proteins present in the viral envelope. Since membrane proteins are very difficult to produce in large amounts, these viral proteins are usually modified to make a soluble form of the proteins. These viral envelope proteins are critical for establishing host immunity, but many attempts to express them in whole or part in heterologous systems have met with limited success, presumably because the protein must be presented to the immune system in the context of a viral envelope membrane in order to be sufficiently immunogenic. Thus, there is a need for new heterologous expression systems, such as those of the present invention, that are scalable and able to present viral antigens free or substantially free of associated viral material, such as viral genetic material, other than the desired viral antigens. The term "substantially free of associated viral material" as used herein means less than 10%, less than 9%, less than 8%, less than 7%, less than 5%, less than 4%, less than 3%, less than 2%, or less than 1% of associated viral material.

In some instances, a microalgal extracellular body comprises a heterologous polypeptide that is a viral glycoprotein selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), a gp41 (glycoprotein of 41 kDa), and combinations thereof. In some instances, the microalgal extracellular body comprises a heterologous polypeptide that is a viral matrix protein. In some instances, the microalgal extracellular body comprises a viral matrix protein selected from the group consisting of M1, M2 (a membrane channel protein), Gag, and combinations thereof. In some instances, the microalgal extracellular body comprises a combination of two or more viral proteins selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), a gp41 (glycoprotein of 41 kDa), and a viral matrix protein.

In some instances, the microalgal extracellular bodies of the present disclosure comprise viral glycoproteins lacking sialic acid that might otherwise interfere with protein accumulation or function.

In some instances, the microalgal extracellular body is a VLP.

The term "VLP" as used herein refers to particles that are morphologically similar to infectious virus that can be formed by spontaneous self-assembly of viral proteins when the viral proteins are over-expressed. VLPs have been produced in yeast, insect, and mammalian cells and appear to be an effective and safer type of subunit vaccine, because they mimic the overall structure of virus particles without containing infectious genetic material. This type of vaccine delivery system has been successful in stimulating the cellular and humoral responses.

Studies on Pararmyxoviruses have shown that when multiple viral proteins were co-expressed, the VLPs produced were very similar in size and density to authentic virions. Expression of the matrix protein (M) alone was necessary and sufficient for VLP formation. In Paramyxovirus, the expression of HN alone resulted in very low efficiency of VLP formation. Other proteins alone were not sufficient for NDV budding. HN is a type II membrane glycoprotein that exists on virion and infected-cell surfaces as a tetrameric spike. *See,* for example, Collins PL and Mottet G, J. Virol. 65:2362-2371 (1991); Mirza AM et al., J. Biol. Chem. 268: 21425-21431 (1993); and Ng D et al., J. Cell. Biol. 109: 3273-3289 (1989). Interactions with the M protein were responsible for incorporation of the proteins HN and NP into VLPs. *See,* for example, Pantua et. al., J. Virology 80:11062-11073 (2006).

Hepatitis B virus (HBV) or the human papillomavirus (HPV) VLPs are simple VLPs that are non-enveloped and that are produced by expressing one or two capsid proteins. More complex non-enveloped VLPs include particles such as VLPs developed for blue-tongue disease. In that case, four of the major structural proteins from the blue-tongue virus (BTV, *Reoviridae* family) were expressed simultaneously in insect cells. VLPs from viruses with lipid envelopes have also been produced (e.g., hepatitis C and influenza A). There are also VLP-like structures such as the self-assembling polypeptide nanoparticles (SAPN) that can repetitively display antigenic epitopes. These have been used to design a potential malaria vaccine. *See,* for example, Kaba SA et al., J. Immunol. 183 (11): 7268-7277 (2009).

VLPs have significant advantages in that they have the potential to generate immunity comparable to live attenuated or inactivated viruses, are believed to be highly immunogenic because of their particulate nature, and because they display surface epitopes in a dense repetitive array. For example, it has been hypothesized that B cells specifically recognize particulate antigens with epitope spacing of 50 Å to 100 Å as foreign. *See* Bachman et al., Science 262: 1448 (1993). VLPs also have a particle size that is believed to greatly facilitate uptake by dendritic cells and macrophages. In addition, particles of 20 nm to 200 nm diffuse freely to lymph nodes, while particles of 500 to 2000 nm do not. There are at least two approved VLP vaccines in humans, Hepatitis B Vaccine (HBV) and Human Papillomavirus (HPV). However, viral-based VLPs such as baculovirus-based VLPs often contain large amounts of viral material that require further purification from the VLPs.

In some instances, the microalgal extracellular body is a VLP comprising a viral glycoprotein selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), a gp41 (glycoprotein of 41 kDa), and combinations thereof. In some instances, the microalgal extracellular body is a VLP comprising a viral matrix protein. In some instances, the microalgal extracellular body is a VLP comprising a viral matrix protein selected from the group consisting of M1, M2 (a membrane channel protein), Gag, and combinations thereof. In some instances, the microalgal extracellular body is a VLP comprising a combination of two or more viral proteins selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), a gp41 (glycoprotein of 41 kDa), and a viral matrix protein.

### Methods of Using the Microalgal Extracellular Bodies

In some instances, a microalgal extracellular body of the disclosure is useful as a vehicle for a protein activity or function. In some instances, the protein activity or function is associated with a heterologous polypeptide present in or on the extracellular body. In some instances, the heterologous polypeptide is a membrane protein. In some instances, the protein activity or function is associated with a polypeptide that binds to a membrane protein present in the extracellular body. In some instances, the protein is not functional when soluble but is functional when part of an extracellular body of the disclosure. In some instances, a microalgal extracellular body containing a sugar transporter (such as, for example, a xylose, sucrose, or glucose transporter) can be used to deplete media containing mixes of sugars or other low molecular weight solutes, of trace amounts of a sugar by capturing the sugar within the vesicles that can then be separated by various methods including filtration or centrifugation.

The present disclosure also includes the use of any of the microalgal extracellular bodies of the disclosure comprising a heterologous polypeptide, and compositions thereof, for therapeutic applications in animals or humans ranging from preventive treatments to disease.

The terms "treat" and "treatment" refer to both therapeutic treatment and prophylactic or preventative measures, wherein the object is to prevent or slow down (lessen) an undesired physiological condition, disease, or disorder, or to obtain beneficial or desired clinical results. For purposes of this invention, beneficial or desired clinical results include, but are not limited to, alleviation or elimination of the symptoms or signs associated with a condition, disease, or disorder; diminishment of the extent of a condition, disease, or disorder; stabilization of a condition, disease, or disorder, (i.e., where the condition, disease, or disorder is not worsening); delay in onset or progression of the condition, disease, or disorder; amelioration of the condition, disease, or disorder; remission (whether partial or total and whether detectable or undetectable) of the condition, disease, or disorder; or enhancement or improvement of a condition, disease, or disorder. Treatment includes eliciting a clinically significant response without excessive side effects. Treatment also includes prolonging survival as compared to expected survival if not receiving treatment.

In some instances, any of the microalgal extracellular bodies of the disclosure comprising a heterologous polypeptide are recovered in the culture supernatant for direct use as animal or human vaccine.

In some instances, a microalgal extracellular body comprising a heterologous polypeptide is purified according to the requirements of the use of interest, e.g., administration as a vaccine. For a typical human vaccine application, the low speed supernatant would undergo an initial purification by concentration (e.g., tangential flow filtration followed by ultrafiltration), chromatographic separation (e.g., anion-exchange chromatography), size exclusion chromatography, and sterilization (e.g., 0.2 µm filtration). In some instances, a vaccine of the disclosure lacks potentially allergenic carry-over proteins such as, for example, egg protein. In some instances, a vaccine comprising an extracellular body of the disclosure lacks any viral material other than a viral polypeptide associated with the extracellular body.

According to the disclosed methods, a microalgal extracellular body comprising a heterologous polypeptide, or a composition thereof, can be administered, for example, by intramuscular (i.m.), intravenous (i.v.), subcutaneous (s.c.), or intrapulmonary routes. Other suitable routes of administration include, but are not limited to intratracheal, transdermal, intraocular, intranasal, inhalation, intracavity, intraductal (e.g., into the pancreas), and intraparenchymal (e.g., into any tissue) administration. Transdermal delivery includes, but is not limited to, intradermal (e.g., into the dermis or epidermis), transdermal (e.g., percutaneous), and transmucosal administration (e.g., into or through skin or mucosal tissue). Intracavity administration includes, but is not limited to, administration into oral, vaginal, rectal, nasal, peritoneal, and intestinal cavities, as well as, intrathecal (e.g., into spinal canal), intraventricular (e.g., into the brain ventricles or the heart ventricles), intraatrial (e.g., into the heart atrium), and subarachnoid (e.g., into the subarachnoid spaces of the brain) administration.

In some instances, the disclosure includes compositions comprising a microalgal extracellular body that comprises a heterologous polypeptide. In some instances, the composition comprises an aqueous liquid carrier. In further instances, the aqueous liquid carrier is a culture supernatant. In some instances, the compositions of the disclosure include conventional pharmaceutically acceptable excipients known in the art such as, but not limited to, human serum albumin, ion exchangers, alumina, lecithin, buffer substances such as phosphates, glycine, sorbic acid, potassium sorbate, and salts or electrolytes such as protamine sulfate, as well as excipients listed in, for example, Remington: The Science and Practice of Pharmacy, 21st ed. (2005).

The most effective mode of administration and dosage regimen for the compositions of this disclosure depends upon the severity and course of the disease, the subject's health and response to treatment and the judgment of the treating physician. Accordingly, the dosages of the compositions should be titrated to the individual subject. Nevertheless, an effective dose of the compositions of this disclosure can be in the range of from 1 mg/kg to 2000 mg/kg, 1 mg/kg to 1500 mg/kg, 1 mg/kg to 1000 mg/kg, 1 mg/kg to 500 mg/kg, 1 mg/kg to 250 mg/kg, 1 mg/kg to 100 mg/kg, 1 mg/kg to 50 mg/kg, 1 mg/kg to 25 mg/kg, 1 mg/kg to 10 mg/kg, 500 mg/kg to 2000 mg/kg, 500 mg/kg to 1500 mg/kg, 500 mg/kg to 1000 mg/kg, 100 mg/kg to 2000 mg/kg, 100 mg/kg to 1500 mg/kg, 100 mg/kg to 1000 mg/kg, or 100 mg/kg to 500 mg/kg.

Having generally described this invention, a further understanding can be obtained by reference to the examples provided herein. These examples are for purposes of illustration only and are not intended to be limiting.

### EXAMPLE 1

### Construction of the pCL0143 expression vector

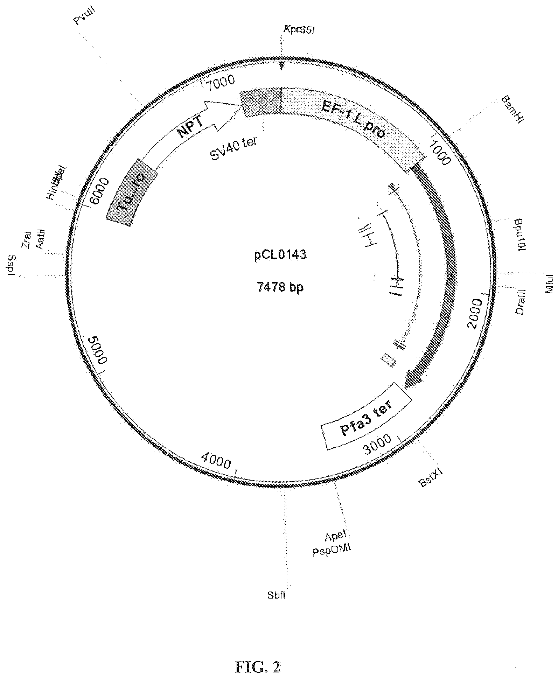

The pCL0143 expression vector (**FIG. 2**) was synthesized and the sequence was verified by Sanger sequencing by DNA 2.0 (Menlo Park, CA). The pCL0143 vector includes a promoter from the *Schizochytrium* elongation factor-1 gene (EF1) to drive expression of the HA transgene, the OrfC terminator (also known as the PFA3 terminator) following the HA transgene, and a selection marker cassette conferring resistance to the antibiotic paromomycin.

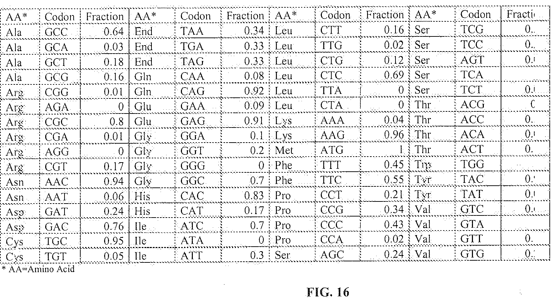

SEQ ID NO: 76 (**FIG. 1**) encodes the HA protein of Influenza A virus (A/Puerto Rico/8/34/Mount Sinai (H1N1)). The protein sequence matches that of GenBank Accession No. AAM75158. The specific nucleic acid sequence of SEQ ID NO: 76 was codon-optimized and synthesized for expression in *Schizochytrium* by DNA 2.0 as guided by the *Schizochytrium* codon usage table shown in **FIG. 16****.** A construct was also produced using an alternative signal peptide in which the signal peptide of SEQ ID NO: 76 (first 51 nucleotides) was removed and replaced by the polynucleotide sequence encoding the *Schizochytrium* Sec1 signal peptide (SEQ ID NO: 38).

### EXAMPLE 2

### Expression and Characterization of HA Protein Produced in Schizochytrium

*Schizochytrium* sp. ATCC 20888 was used as a host cell for transformation with the vector pCL0143 with a Biolistic^{™} particle bombarder (BioRad, Hercules, CA). Briefly, cultures of *Schizochytrium* sp. ATCC number 20888 were grown in M2B medium consisting of 10 g/L glucose, 0.8 g/L (NH₄)₂SO₄, 5 g/L Na₂SO₄, 2 g/L MgSO₄•7H₂O, 0.5 g/L KH₂PO₄, 0.5 g/L KCl, 0.1 g/L CaCl₂•2H₂O, 0.1 M MES (pH 6.0), 0.1% PB26 metals, and 0.1% PB26 Vitamins (v/v). PB26 vitamins consisted of 50 mg/mL vitamin B12, 100 µg/mL thiamine, and 100 µg/mL Ca-pantothenate. PB26 metals were adjusted to pH 4.5 and consisted of 3 g/L FeSO₄•7H₂O, 1 g/L MnCl₂•4H₂O, 800 mg/mL ZnSO₄•7H₂O, 20 mg/mL CoCl₂•6H₂O, 10 mg/mL Na₂MoO₄•2H₂O, 600 mg/mL CuSO₄•5H₂O, and 800 mg/mL NiSO₄•6H₂O. PB26 stock solutions were filter-sterilized separately and added to the broth after autoclaving. Glucose, KH₂PO₄, and CaCl₂•2H₂O were each autoclaved separately from the remainder of the broth ingredients before mixing to prevent salt precipitation and carbohydrate caramelizing. All medium ingredients were purchased from Sigma Chemical (St. Louis, MO). Cultures of *Schizochytrium* were grown to log phase and transformed with a Biolistic^{™} particle bombarder (BioRad, Hercules, CA). The Biolistic^{™} transformation procedure was essentially the same as described previously (see Apt et al., J. Cell. Sci. 115(Pt 21):4061-9 (1996) and U.S. Patent No. 7,001,772). Primary transformants were selected on solid M2B media containing 20 g/L agar (VWR, West Chester, PA), 10 µg/mL Sulfometuron methyl (SMM) (Chem Service, Westchester, PA) after 2-6 days of incubation at 27°C.

gDNA from primary transformants of pCL0143 was extracted and purified and used as a template for PCR to check for the presence of the transgene.

*Genomic DNA Extraction Protocol for Schizochytrium* - The *Schizochytrium* transformants were grown in 50 ml of media. 25 ml of culture was aseptically pipetted into a 50 ml conical vial and centrifuge for 4 minutes at 3000 × g to form a pellet. The supernatant was removed and the pellet stored at -80°C until use. The pellet was resuspended in approximately 4-5 volumes of a solution consisting of 20 mM Tris pH 8, 10 mM EDTA, 50 mM NaCl, 0.5% SDS and 100 µg/ml of Proteinase K in a 50 ml conical vial. The pellet was incubated at 50°C with gentle rocking for 1 hour. Once lysed, 100 µg/ml of RNase A was added and the solution was rocked for 10 minutes at 37° C. Next, 2 volumes of phenol: chloroform: isoamyl alcohol was added and the solution was rocked at room temperature for 1 hour and then centrifuged at 8000 × g for 15 minutes. The supernatant was transferred into a clean tube. Again, 2 volumes of phenol: chloroform :isoamyl alcohol was added and the solution was rocked at room temperature for 1 hour and then centrifuged at 8000 × g for 15 minutes and the supernatant was transferred into a clean tube. An equal volume of chloroform was added to the resulting supernatant and the solution was rocked at room temperature for 30 minutes. The solution was centrifuged at 8000 × g for 15 minutes and the supernatant was transferred into a clean tube. An equal volume of chloroform was added to the resulting supernatant and the solution was rocked at room temperature for 30 minutes. The solution was centrifuged at 8000 × g for 15 minutes and the supernatant was transferred into a clean tube. 0.3 volumes of 3M NaOAc and 2 volumes of 100% EtOH were added to the supernatant, which was rocked gently for a few minutes. The DNA was spooled with a sterile glass rod and dipped into 70 % EtOH for 1-2 minutes. The DNA was transferred into a 1.7 ml microfuge tube and allowed to air dry for 10 minutes. Up to 0.5 ml of pre-warmed EB was added to the DNA and it was placed at 4° C overnight.

Cryostocks of transgenic *Schizochytrium* (transformed with pCL0143) were grown in M50-20 to confluence and then propagated in 50 mL baffled shake flasks at 27°C, 200 rpm for 48 hours (h), unless indcated otherwise, in a medium containing the following (per liter):

| | |
|---|---|
| Na₂SO₄ | 13.62g |
| K₂SO₄ | 0.72g |
| KCl | 0.56g |
| MgSO₄.7H₂O | 2.27g |
| (NH₄)2SO₄ | 3g |
| CaCl₂.2H₂O | 0.19g |
| MSG monohydrate | 3g |
| MES | 21.4g |
| KH₂PO₄ | 0.4g |

The volume was brought to 900 mL with deionized H₂O and the pH was adjusted to 6.5, unless indicated otherwise, before autoclaving for 35 min. Filter-sterilized glucose (50 g/L), vitamins (2 mL/L) and trace metals (2 mL/L) were then added to the medium and the volume was adjusted to one liter. The vitamin solution contained 0.16 g/L vitamin B12, 9.75 g/L thiamine, and 3.33 g/L Ca-pentothenate. The trace metal solution (pH 2.5) contained 1.00 g/L citric acid, 5.15 g/L FeSO₄.7H₂O, 1.55 g/L MnCl₂.4H₂O, 1.55 g/L ZnSO₄.7H₂O, 0.02 g/L CoCl₂.6H₂O, 0.02 g/L Na₂MoO₄.2H₂O, 1.035 g/L CuSO₄.5H₂O, and 1.035 g/L NiSO₄.6H₂O.

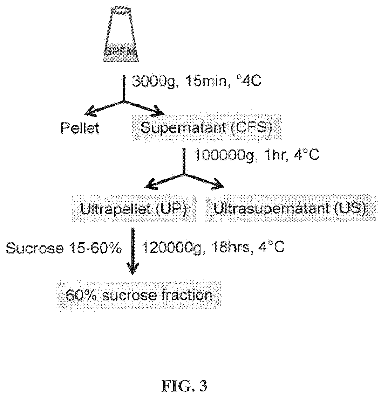

*Schizochytrium* cultures were transferred to 50 mL conical tubes and centrifugated at 3000 × g or 4500 × g for 15 min. *See* **FIG. 3****.** The supernatant resulting from this centrifugation, termed the "cell-free supernatant" (CFS), was used for a immunoblot analysis and a hemagglutination activity assay.

The cell-free supernatant (CFS) was further ultracentrifugated at 100,000 × g for 1 h. *See* **FIG. 3****.** The resulting pellet (insoluble fraction or "UP") containing the HA protein was resuspended in PBS, pH 7.4. This suspension was centrifuged (120,000 × g, 18 h, 4°C) on a discontinuous sucrose density gradient containing sucrose solutions from 15-60%. *See* **FIG. 3****.** The 60% sucrose fraction containing the HA protein was used for peptide sequence analysis, glycosylation analysis, as well as electron microscopy analysis.

### Immunoblot Analysis

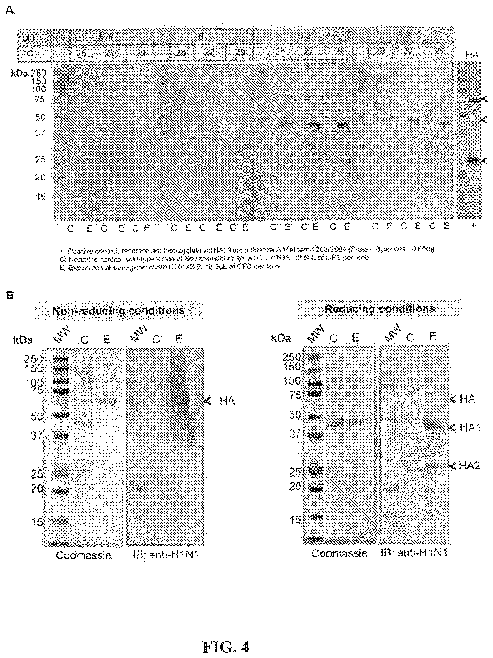

The expression of the recombinant HA protein from transgenic Schizochytrium CL0143-9 ("E") was verified by immunoblot analysis following standard immunoblotting procedure. The proteins from the cell-free supernatant (CFS) were separated by sodium dodecyl sulfate-polyacrylamide gel electrophoresis (SDS-PAGE) on a NuPAGE^{®} Novex^{®} 12% bis-tris gel (Invitrogen, Carlsbad, CA) under reducing conditions with MOPS SDS running buffer, unless indicated otherwise. The proteins were then stained with Coomassie blue (SimplyBlue Safe Stain, Invitrogen, Carlsbad, CA) or transferred onto polyvinylidene fluoride membrane and probed for the presence of HA protein with anti-Influenza A/Puerto Rico/8/34 (H1N1) virus antiserum from rabbit (1:1000 dilution, gift from Dr. Albert D.M.E. Osterhaus; Fouchier R.A.M. et al., J. Virol. 79: 2814-2822 (2005)) followed by anti-rabbit IgG (Fc) secondary antibody coupled to alkaline phosphatase (1:2000 dilution, #S3731, Promega Corporation, Madison, WI). The membrane was then treated with 5-bromo-4-chloro-3-indoyl-phosphate/nitroblue tetrazolium solution (BCIP/NBT) according to the manufacturer's instructions (KPL, Gaithersburg, MD). Anti-H1N1 immunoblots for the transgenic Schizochytrium CL0143-9 ("E") grown at various pH (5.5, 6.0, 6.5 and 7.0) and various temperatures (25°C, 27°C, 29°C) are shown in FIG. 4A. The negative control ("C") was the wild-type strain of Schizochytrium sp. ATCC 20888. The recombinant HA protein was detected in the cell-free supernatant at pH 6.5 (FIG. 4A) and hemagglutination activity detected was highest at pH 6.5, 27°C (FIG. 4A). Coomassie blue-stained gels ("Coomassie") and corresponding anti-H1N1 immunoblots ("IB: anti-H1N1") for CL0143-9 ("E") grown at pH 6.5, 27°C, are shown in FIG. 4B under non-reducing and reducing conditions. The negative control ("C") was the wild-type strain of *Schizochytrium* sp. ATCC 20888.

### HA Activity

The activity of the HA protein produced in *Schizochytrium* was evaluated by a hemagglutination activity assay. The functional HA protein displays a hemagglutination activity that is readily detected by a standard hemagglutination activity assay. Briefly, 50 µL of doubling dilutions of low speed supernatant in PBS were prepared in a 96-well microtiter plate. Equal volume of an approximate 1% solution of chicken red blood cells (Fitzgerald Industries, Acton, MA) in PBS, pH 7.4, was then added to each well followed by incubation at room temperature for 30 min. The degree of agglutination was then analyzed visually. The hemagglutination activity unit (HAU) is defined as the highest dilution that causes visible hemagglutination in the well.

Typical activity was found to be in the order of 512 HAU in transgenic *Schizochytrium* CL0143-9 ("E") cell free supernatant (**FIG. 5A**). PBS ("-") or the wild-type strain of *Schizochytrium* sp. ATCC 20888 ("C"), grown and prepared in the same manner as the transgenic strains, were used as negative controls and did not show any hemagglutination activity. The recombinant HA protein from Influenza A/Vietnam/1203/2004 (H5N1) (Protein Sciences Corporation, Meriden, CT, dilution 1:1000 in PBS) was used as a positive control ("+").

Analysis of the soluble and insoluble fractions of the cell-free supernatant of the transgenic *Schizochytrium* CL0143-9 strain by hemagglutination assay a indicated that the HA protein is found predominantly in the insoluble fraction (**FIG. 5B**). Typical activity was found to be in the order of 16HAU in the soluble fraction ("US") and 256 HAU in the insoluble fraction ("UP").

Activity levels of HA protein in 2 L cultures demonstrated similar activity as in shake flask cultures when cultured in the same media at a constant pH of 6.5.

In a separate experiment, the native signal peptide of HA was removed and replaced by the *Schizochytrium* Sec1 signal peptide (SEQ ID NO: 37, encoded by SEQ ID NO: 38). Transgenic *Schizochytrium* obtained with this alternative construct displayed similar hemagglutin activity and recombinant protein distribution as observed with transgenic *Schizochytrium* containing the pCL0143 construct (data not shown).

### Peptide Sequence Analysis

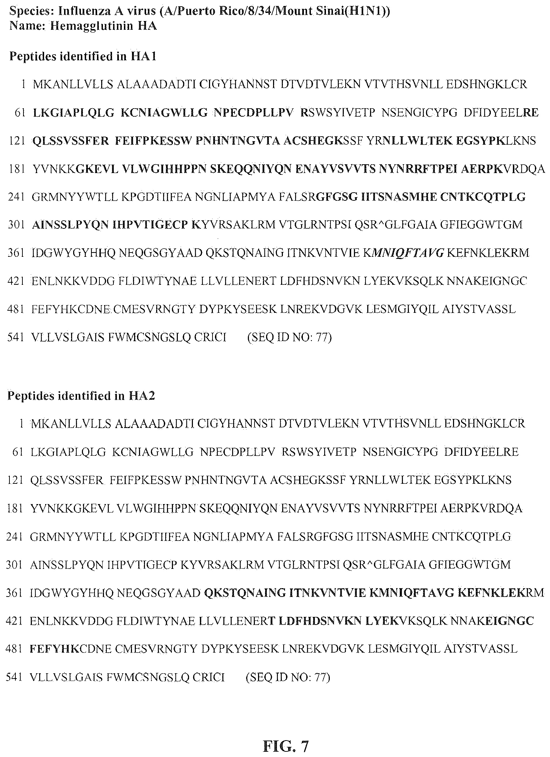

The insoluble fraction ("UP") resulting from 100,000 × g centrifugation of the cell-free supernatant was further fractionated on sucrose density gradient and the fractions containing the HA protein, as indicated by hemagglutination activity assay (FIG. 6B), was separated by SDS-PAGE and stained with Coomassie blue or transferred to PVDF and immunoblotted with anti-H1N1 antiserum from rabbit (FIG. 6A), as described above. The bands corresponding to the cross-reaction in immunoblot (HA1 and HA2) were excised from the Coomassie blue-stained gel and peptide sequence analysis was performed. Briefly, the bands of interest were washed/destained in 50% ethanol, 5% acetic acid. The gel pieces were then dehydrated in acetonitrile, dried in a SpeedVac^{®} (Thermo Fisher Scientific, Inc., Waltham, MA), and digested with trypsin by adding 5 µL of 10 ng/µL trypsin in 50 mM ammonium bicarbonate and incubating overnight at room temperature. The peptides that were formed were extracted from the polyacrylamide in two aliquots of 30 µL 50% acetonitrile with 5% formic acid. These extracts were combined and evaporated to <10 µL in a SpeedVac^{®} and then resuspended in 1% acetic acid to make up a final volume of approximately 30 µL for LC-MS analysis. The LC-MS system was a FinniganTM LTQTM Linear Ion Trap Mass Spectrometer (Thermo Electron Corporation, Waltham, MA). The HPLC column was a self-packed 9 cm × 75 µm Phenomenex JupiterTM C18 reversed-phase capillary chromatography column (Phenomenex, Torrance, CA). Then, µL volumes of the extract were injected and the peptides were eluted from the column by an acetonitrile/0.1% formic acid gradient at a flow rate of 0.25 µL/min and were introduced into the source of the mass spectrometer on-line. The microelectrospray ion source was operated at 2.5 kV. The digest was analyzed using a selective reaction (SRM) experiment in which the mass spectrometer fragments a series of m/z ratios over the entire course of the LC experiment. The fragmentation pattern of the peptides of interest was then used to produce chromatograms. The peak areas for each peptide was determined and normalized to an internal standard. The internal standards used in this analysis were proteins that have an unchanging abundance between the samples being studied. The final comparison between the two systems was determined by comparing the normalized peak ratios for each protein. The collision-induced dissociation spectra were then searched against the NCBI database. The HA protein was identified by a total of 27 peptides covering over 42% of the protein sequence. The specific peptides that were sequenced are highlighted in bold font in FIG. 7. More specifically, HA1 was identified by a total of 17 peptides and HA2 was identified by a total of 9 peptides. This is consistent with the HA N-terminal polypeptide being truncated prior to position 397. The placement of the identified peptides for HA1 and HA2 are shown within the entire amino acid sequence of the HA protein. The putative cleavage site within HA is located between amino acids 343 and 344 (shown as R^G). The italicized peptide sequence beginning at amino acid 402 is associated with the HA2 polypeptide but appeared in the peptides identified in HA1, likely due to trace carryover of HA2 peptides in the excised band for HA1. See, for example, Figure 3 of Wright et al., BMC Genomics 10:61 (2009).

### Glycosylation Analysis

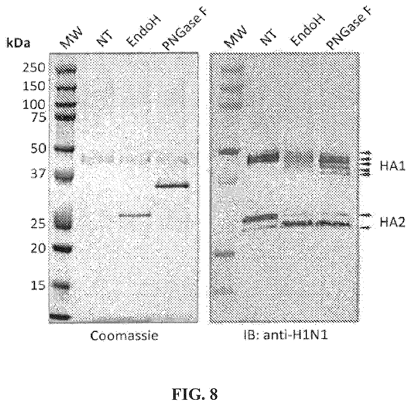

The presence of glycans on the HA protein was evaluated by enzymatic treatment. The 60% sucrose fraction of the transgenic *Schizochytrium* "CL0143-9" was digested with EndoH or PNGase F according to manufacturer's instructions (New England Biolabs, Ipswich, MA). Removal of glycans was then identified by the expected shift in mobility when separating the proteins by SDS-PAGE on NuPAGE^{®} Novex^{®} 12% bis-tris gels (Invitrogen, Carlsbad, CA) with MOPS SDS running buffer followed by staining with Coomassie blue ("Coomassie") or by immunoblotting with anti-H1N1 antiserum ("IB: anti-H1N1") (**FIG. 8**). The negative control for the enzymatic treatment was the transgenic *Schizochytrium* "CL0143-9" incubated without enzymes ("NT" = non-treated). At least five different species can be identified on the immunoblot at the level of HA1 and two different species can be identified on the immunoblot at the level of HA2. This is consistent with multiple glycosylation sites on HA1 and a single glycosylation site on HA2, as reported in the literature.

### EXAMPLE 3

### Characterization of Proteins from Schizochytrium Culture Supernatants

*Schizochytrium* sp. ATCC 20888 was grown under typical fermentation conditions as described above. Samples of culture supernatant were collected in 4 hour intervals from 20 h to 52 h of culture, with a final collection at 68 h.

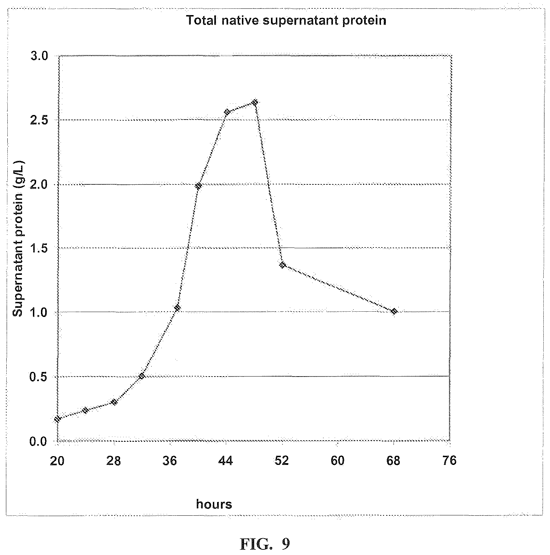

Total protein in the culture supernatant based on each sample was determined by a standard Bradford Assay. *See* **FIG. 9****.**

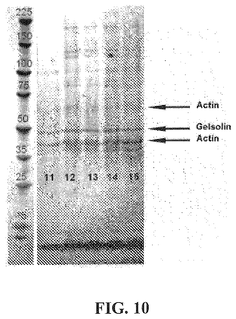

Proteins were isolated from the samples of culture supernatant at 37 h, 40 h, 44 h, 48 h, and 68 h using the method of **FIG. 3****.** A SDS-PAGE gel of the proteins is shown in **FIG. 10****.** Lane 11 was loaded with 2.4 µg of total protein, the remaining lanes were loaded with 5 µg total protein. Abundant bands identified as actin or gelsolin (by mass spectral peptide sequencing) are marked with arrows in **FIG. 10****.**

### EXAMPLE 4

### Negative-Staining and Electron Microscopy of Culture Supernatant Materials

*Schizochytrium* sp. ATCC 20888 (control) and transgenic *Schizochytrium* CL0143-9 (experimental) were grown under typical flask conditions as described above. Cultures were transferred to 50 mL conical tubes and centrifugated at 3000 × g or 4500 × g for 15 min. This cell-free supernatant was further ultracentrifugated at 100,000 × g for 1 h and the pellet obtained was resuspended in PBS, pH 7.4. This suspension was centrifuged on a discontinuous 15% to 60% sucrose gradient (120,000 × g, 18 h, 4°C), and the 60% fraction was used for negative-staining and examination by electron microscopy.

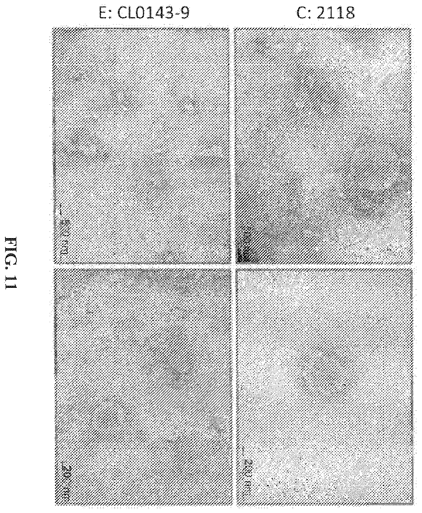

Electron microscope observations of control material negative-stained material contained a mixture of membrane fragments, membrane aggregates and vesicles (collectively "extracellular bodies") ranging from hundreds of nanometers in diameter to <50 nm. *See* **FIG. 11****.** Vesicle shape ranged from circular to elongated (tubular), and the margins of the vesicles were smooth or irregular. The interior of the vesicles appeared to stain lightly, suggesting that organic material was present. The larger vesicles had thickened membranes, suggesting that edges of the vesicles overlapped during preparation. Membrane aggregates and fragments were highly irregular in shape and size. The membrane material likely originated from the ectoplasmic net, as indicated by a strong correlation with actin in membranes purified by ultracentrifugation.

Similarly, electron microscope observations of negative-stained material from cell-free supernatants of culture of transgenic *Schizochytrium* CL0143-9 expressing heterologous protein indicated that the material was a mixture of membrane fragments, membrane aggregates and vesicles ranging from hundreds of nanometers in diameter to <50 nm. *See* **FIG. 11****.**

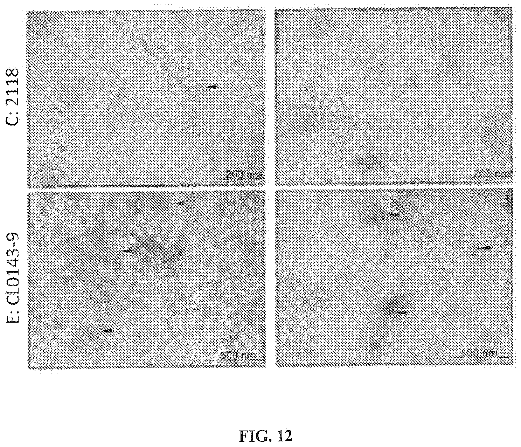

Immunolocalization was also conducted on this material as described in Perkins et al., J. Virol. 82:7201-7211 (2008), using the H1N1 antiserum described for the immunoblot analysis in Example 22 and 12 nm gold particles. Extracellular membrane bodies isolated from transgenic *Schizochytrium* CL0143-9 were highly decorated by gold particles attached to the antiserum (**FIG. 12**), indicating that the antibody recognized HA protein present in the extracellular bodies. Minimal background was observed in areas absent of membrane material. There were few or no gold particles bound to extracellular bodies isolated from control material (**FIG. 12**).

### EXAMPLE 5

### Construction of Xylose Transporter, Xylose Isomerase and Xylulose Kinase Expression Vectors

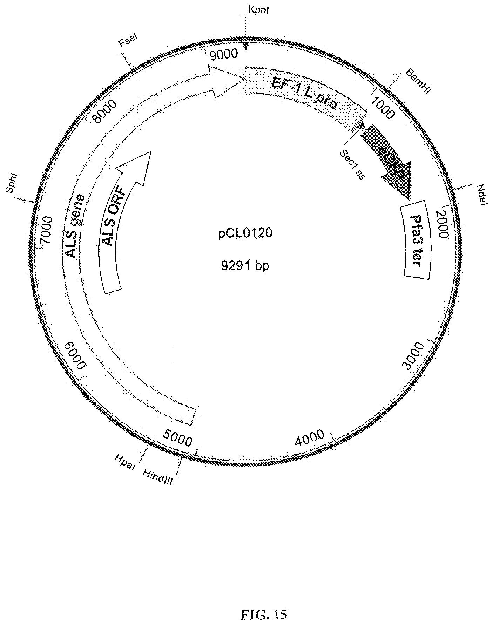

The vector pAB0018 (ATCC Accession No. PTA-9616) was digested with HindIII, treated with mung bean nuclease, purified, and then further digested with KpnI generating four fragments of various sizes. A fragment of 2552 bp was isolated by standard electrophoretic techniques in an agar gel and purified using commercial DNA purification kits. A second digest of pAB0018 with PmeI and Kpn was then performed. A fragment of 6732 bp was isolated and purified from this digest and ligated to the 2552 bp fragment. The ligation product was then used to transform commercially supplied strains of competent DH5-α *E. coli* cells (Invitrogen) using the manufacturer's protocol. Plasmids from ampicillin-resistant clones were propagated, purified, and then screened by restriction digests or PCR to confirm that the ligation generated the expected plasmid structures. One verified plasmid was designated pCL0120. *See* **FIG. 15****.**

Sequences encoding the *Candida intermedia* xylose transporter protein GXS1 (GenBank Accession No. AJ875406) and the *Arabidopsis thaliana* xylose transporter protein At5g17010 (GenBank Accession No. BT015128) were codon-optimized and synthesized (Blue Heron Biotechnology, Bothell, WA) as guided by the *Schizochytrium* codon usage table shown in **FIG. 16****.** SEQ ID NO: 94 is the codon-optimized nucleic acid sequence of GSX1, while SEQ ID NO: 95 is the codon-optimized nucleic acid sequence of At5g17010.

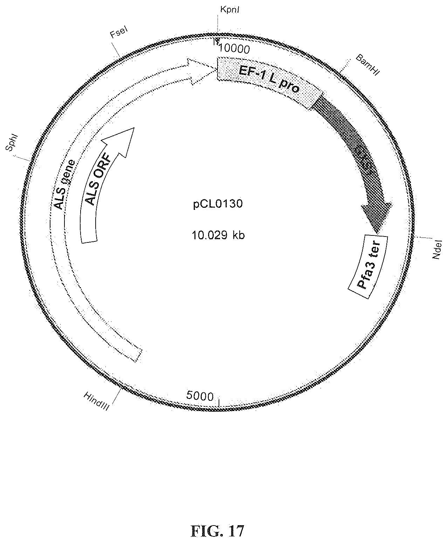

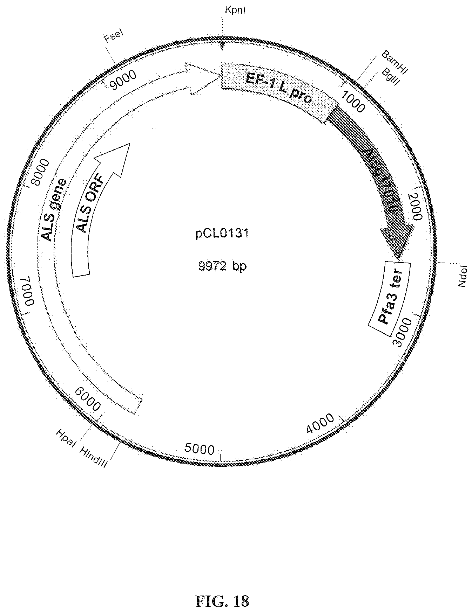

SEQ ID NO: 94 and SEQ ID NO: 95 were respectively cloned into pCL0120 using the 5' and 3' restriction sites BamHI and NdeI for insertion and ligation according to standard techniques. Maps of the resulting vectors, pCL0130 and pCL0131 are shown in **FIG. 17** and **FIG. 18****,** respectively.

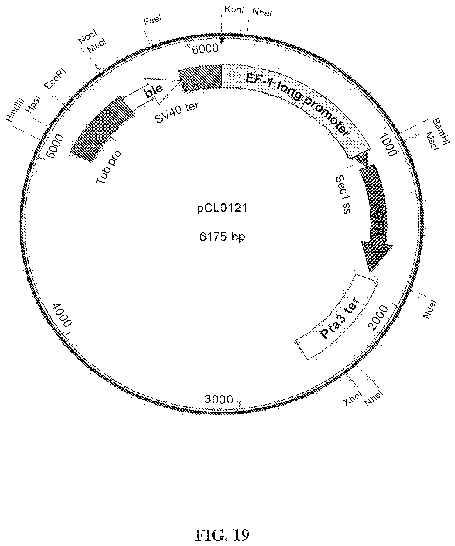

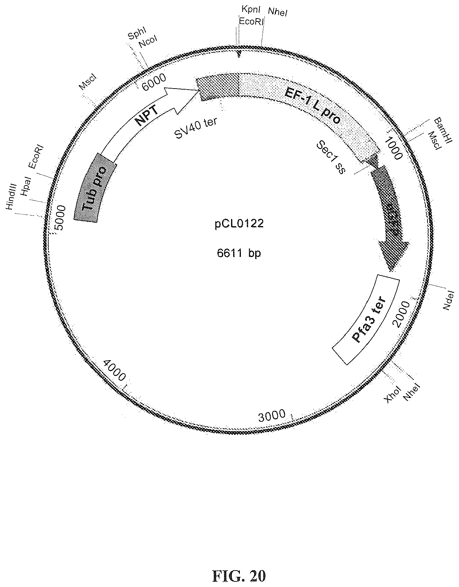

Vectors pCL0121 and pCL0122 were created by ligating a 5095 bp fragment which had been liberated from pCL0120 by digestion with HindIII and KpnI to synthetic selectable marker cassettes designed to confer resistance to either zeocin or paromomycin. These cassettes were comprised of an alpha tubulin promoter to drive expression of either the sh ble gene (for zeocin) or the npt gene (for paromomycin). The transcripts of both selectable marker genes were terminated by an SV40 terminator. The full sequence of vectors pCL0121 and pCL0122 are provided as SEQ ID NO: 90 and SEQ ID NO: 91, respectively. Maps of vectors pCL0121 and pCL0122 are shown in **FIGs. 19** and **20****,** respectively.

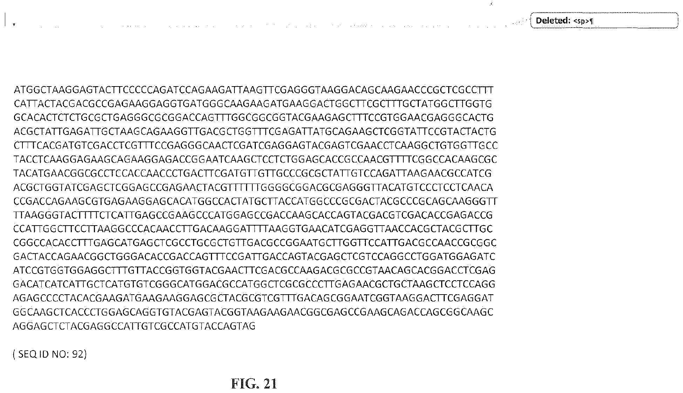

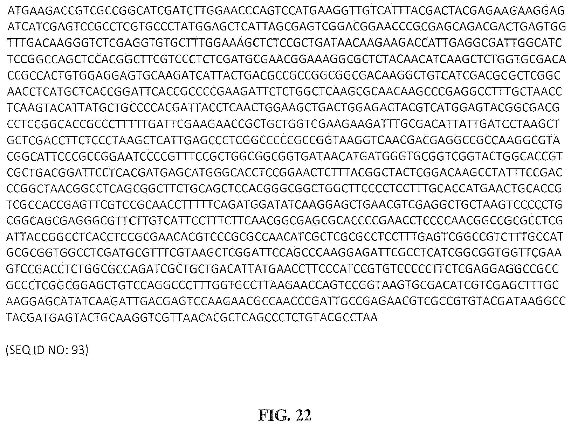

Sequences encoding the *Piromyces* sp. E2 xylose isomerase (CAB76571) and *Piromyces* sp. E2 xylulose kinase (AJ249910) were codon-optimized and synthesized (Blue Heron Biotechnology, Bothell, WA) as guided by the *Schizochytrium* codon usage table shown in **FIG. 16****.** "XylA" (SEQ ID NO: 92) is the codon-optimized nucleic acid sequence of CAB76571 (**FIG. 21**), while "XylB" (SEQ ID NO: 93) is the codon-optimized nucleic acid sequence of AJ249910 (**FIG. 22**).

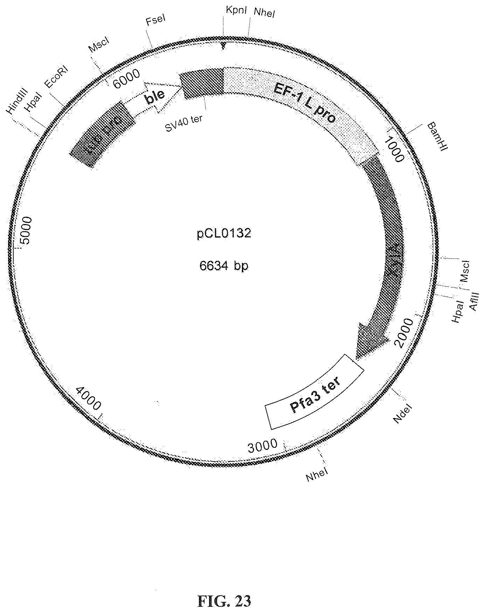

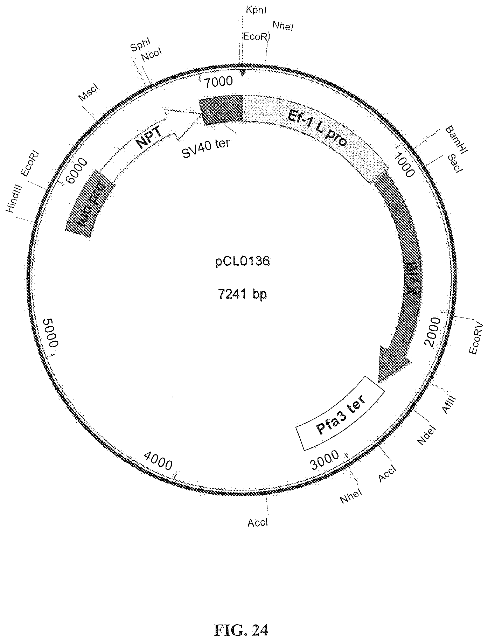

SEQ ID NO: 92 was cloned into the vector pCL0121 resulting in the vector designated pCL0132 (**FIG. 23**) and SEQ ID NO: 21 was cloned into the vector pCL0122 by insertion into the BamHI and NdeI sites, resulting in the vector designated pCL0136 (**FIG. 24**).

### EXAMPLE 6

### Expression and Characterization of Xylose Transporter, Xylose Isomerase and Xylulose Kinase Proteins Produced in Schizochytrium

*Schizochytrium* sp. ATCC 20888 was used as a host cell for transformation with vector pCL0130, pCL0131, pCL0132 or pCL0136 individually.

*Electroporation with enzyme pretreatment* - Cells were grown in 50 mL of M50-20 media (*see* U.S. Publ. No. 2008/0022422) on a shaker at 200 rpm for 2 days at 30°C. The cells were diluted at 1:100 into M2B media (see following paragraph) and grown overnight (16-24 h), attempting to reach mid-log phase growth (OD₆₀₀ of 1.5-2.5). The cells were centrifuged in a 50 mL conical tube for 5 min at 3000 x g. The supernatant was removed and the cells were resuspended in 1 M mannitol, pH 5.5, in a suitable volume to reach a final concentration of 2 OD₆₀₀ units. 5 mL of cells were aliquoted into a 25 mL shaker flask and amended with 10 mM CaCl₂ (1.0 M stock, filter sterilized) and 0.25 mg/mL Protease XIV (10 mg/mL stock, filter sterilized; Sigma-Aldrich, St. Louis, MO). Flasks were incubated on a shaker at 30°C and 100 rpm for 4 h. Cells were monitored under the microscope to determine the degree of protoplasting, with single cells desired. The cells were centrifuged for 5 min at 2500 × g in round-bottom tubes (i.e., 14 mL Falcon^{™} tubes, BD Biosciences, San Jose, CA). The supernatant was removed and the cells were gently resuspended with 5 mL of ice cold 10% glycerol. The cells were re-centrifuged for 5 min at 2500 × g in round-bottom tubes. The supernatant was removed and the cells were gently resuspended with 500 µL of ice cold 10% glycerol, using wide-bore pipette tips. 90 µL of cells were aliquoted into a prechilled electro-cuvette (Gene Pulser^{®} cuvette - 0.2 cm gap, Bio-Rad, Hercules, CA). 1 µg to 5 µg of DNA (in less than or equal to a 10 µL volume) was added to the cuvette, mixed gently with a pipette tip, and placed on ice for 5 min. Cells were electroporated at 200 ohms (resistance), 25 µF (capacitance), and 500V. 0. 5 mL of M50-20 media was added immediately to the cuvette. The cells were then transferred to 4.5 mL of M50-20 media in a 25 mL shaker flask and incubated for 2-3 h at 30°C and 100 rpm on a shaker. The cells were centrifuged for 5 min at 2500 × g in round bottom tubes. The supernatant was removed and the cell pellet was resuspended in 0.5 mL of M50-20 media. Cells were plated onto an appropriate number (2 to 5) of M2B plates with appropriate selection (if needed) and incubated at 30°C.

M2B media consisted of 10 g/L glucose, 0.8 g/L (NH4)2SO4, 5 g/L Na2SO4, 2 g/L MgSO4•7H2O, 0.5 g/L KH2PO4, 0.5 g/L KCl, 0.1 g/L CaCl2•2H2O, 0.1 M MES (pH 6.0), 0.1% PB26 metals, and 0.1% PB26 Vitamins (v/v). PB26 vitamins consisted of 50 mg/mL vitamin B12, 100 µg/mL thiamine, and 100 µg/mL Ca-pantothenate. PB26 metals were adjusted to pH 4.5 and consisted of 3 g/L FeSO4•7H2O, 1 g/L MnCl2•4H2O, 800 mg/mL ZnSO4•7H2O, 20 mg/mL CoCl2•6H2O, 10 mg/mL Na2MoO4•2H2O, 600 mg/mL CuSO4•5H2O, and 800 mg/mL NiSO4•6H2O. PB26 stock solutions were filter-sterilized separately and added to the broth after autoclaving. Glucose, KH2PO4, and CaCl2•2H2O were each autoclaved separately from the remainder of the broth ingredients before mixing to prevent salt precipitation and carbohydrate caramelizing. All medium ingredients were purchased from Sigma Chemical (St. Louis, MO).

The transformants were selected for growth on solid media containing the appropriate antibiotic. Between 20 and 100 primary transformants of each vector were re-plated to "xylose-SSFM" solid media which is the same as SSFM (described below) except that it contains xylose instead of glucose as a sole carbon source, and no antibiotics were added. No growth was observed for any clones under these conditions.

SSFM media: 50 g/L glucose, 13.6 g/L Na₂SO₄, 0.7 g/L K₂SO₄, 0.36 g/L KCl, 2.3 g/L MgSO₄•7H₂O, 0.1M MES (pH 6.0), 1.2 g/L (NH₄)₂SO₄, 0.13 g/L monosodium glutamate, 0.056 g/L KH₂PO₄, and 0.2 g/L CaCl₂•2H₂O. Vitamins were added at 1 mL/L from a stock consisting of 0.16 g/L vitamin B12, 9.7 g/L thiamine, and 3.3 g/L Ca-pantothenate. Trace metals were added at 2 mL/L from a stock consisting of 1 g/L citric acid, 5.2 g/L FeSO₄•7H₂O, 1 .5 g/L MnCl₂•₄H₂O, 1.5 g/L ZnSO₄•7H₂O, 0.02 g/L CaCl₂•6H₂O, 0.02 g/L Na₂MoO₄•2H₂O, 1.0 g/L CuSO₄•5H₂O, and 1.0 g/L NiSO₄•6H₂O, adjusted to pH 2.5.

gDNA from primary transformants of pCL0130 and pCL0131 was extracted and purified and used as a template for PCR to check for the presence of the transgene.

Genomic DNA Extraction was performed as described in Example 2.

Alternatively, after the RNase A incubation, the DNA was further purified using a Qiagen Genomic tip 500/G column (Qiagen, Inc USA, Valencia, CA), following the manufacturers protocol.

*PCR* - The primers used for detecting the GXS1 transgene were 5'CL0130 (CCTCGGGCGGCGTCCTCTT) (SEQ ID NO: 96) and 3'CL0130 (GGCGGCCTTCTCCTGGTTGC) (SEQ ID NO: 97). The primers used for detecting the At5g17010 transgene were 5'CL0131 (CTACTCCGTTGTTGCCGCCATCCT) (SEQ ID NO: 98) and 3'CL0131 (CCGCCGACCATACCGAGAACGA) (SEQ ID NO: 99).

Combinations of pCL0130, pCL0132, and pCL0136 together (the "pCL01310 series") or pCL0131, pCL0132, and pCL0136 together (the "pCL0131 series") were used for co-transformations of *Schizochytrium* wild type strain (ATCC 20888). Transformants were plated directly on solid xylose SSFM media and after 3-5 weeks, colonies were picked and further propagated in liquid xylose-SSFM. Several rounds of serial transfers in xylose-containing liquid media improved growth rates of the transformants. Co-transformants of the pCL0130 series or the pCL0131 series were also plated to solid SSFM media containing either SMM, zeocin, or paromomycin. All transformants plated to these media were resistant to each antibiotic tested, indicating that transformants harbored all three of their respective vectors. The *Schizochytrium* transformed with a xylose transporter, a xylose isomerase and a xylulose kinase were able to grow in media containing xylose as a sole carbon source.

In a future experiment, Western blots of both cell-free extract and cell-free supernatant from shake flask cultures of selected SMM-resistant transformant clones (pCL0130 or pCL0131 transformants alone, or the pCL0130 series co-transformants, or the pCL0131 series co-transformants) are performed and show that both transporters are expressed and found in both fractions, indicating that these membrane-bound proteins are associated with extracellular vesicles in a manner similar to that observed with other membrane proteins described herein. Additionally, Western blots are performed that show expression of the xylose isomerase and xylulose kinase in the cell-free extracts of all clones where their presence is expected. Extracellular bodies such as vesicles containing xylose transporters can be used to deplete media containing mixes of sugars or other low molecular weight solutes, of trace amounts of xylose by capturing the sugar within the vesicles that can then be separated by various methods including filtration or centrifugation.

### EXAMPLE 7

### Construction of the pCL0140 and pCL0149 expression vectors

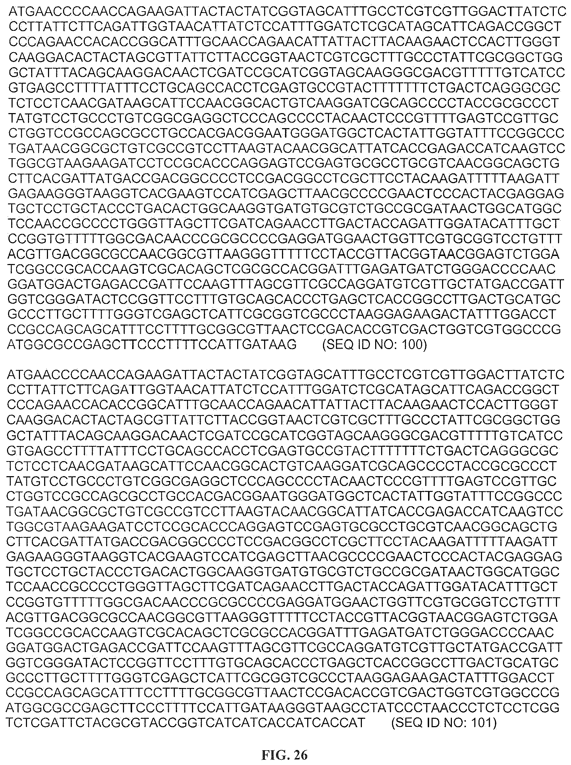

The vector pCL0120 was digested with BamHI and NdeI resulting in two fragments of 837 base pairs (bp) and 8454 bp in length. The 8454 bp fragment was fractionated by standard electrophoretic techniques in an agar gel, purified using commercial DNA purification kits, and ligated to a synthetic sequence (SEQ ID NO: 100 or SEQ ID NO: 101; *see* **FIG. 26**) that had also been previously digested with BamHI and NdeI. SEQ ID NO: 100 (**FIG. 26**) encodes the NA protein of Influenza A virus (A/Puerto Rico/8/34/Mount Sinai(HlNl)). The protein sequence matches that of GenBank Accession No. NP_040981. The specific nucleic acid sequence of SEQ ID NO: 100 was codon-optimized and synthesized for expression in *Schizochytrium* by DNA 2.0 as guided by the *Schizochytrium* codon usage table shown in **FIG. 16**. SEQ ID NO: 101 (**FIG. 26**) encodes the same NA protein as SEQ ID NO: 100, but includes a V5 tag sequence as well as a polyhistidine sequence at the C-terminal end of the coding region.

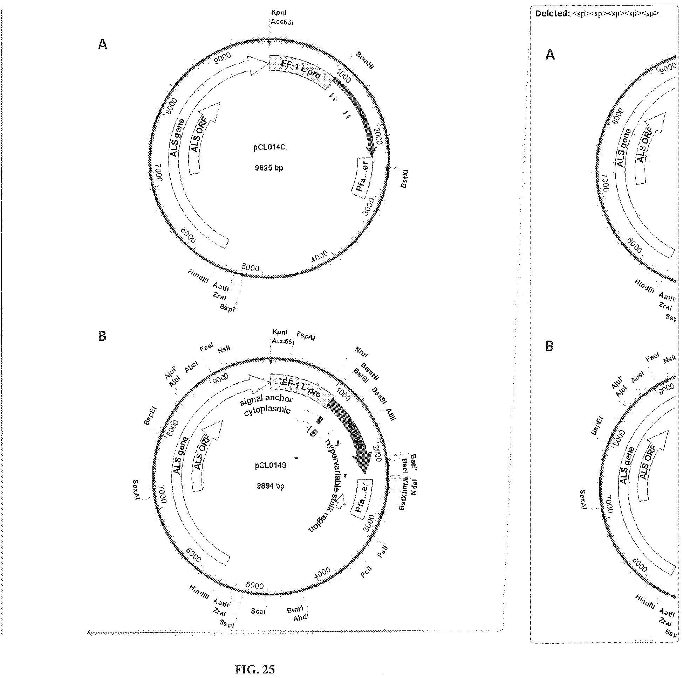

The ligation product was then used to transform commercially supplied strains of competent DH5-α *E*. *coli* cells (Invitrogen, Carlsbad, CA) using the manufacturer's protocol. These plasmids were then screened by restriction digests or PCR to confirm that the ligation generated the expected plasmid structures. Plasmid vectors resulting from the procedure were verified using Sanger sequencing by DNA 2.0 (Menlo Park, CA) and designated pCL0140 (**FIG. 25A**), containing SEQ ID NO: 100, and pCL0149 **(****FIG. 25B****),** containing SEQ ID NO: 101. The pCL0140 and pCL0149 vectors include a promoter from the *Schizochytrium* elongation factor-1 gene (EF1) to drive expression of the NA transgene, the OrfC terminator (also known as the PFA3 terminator) following the NA transgene, and a selection marker cassette conferring resistance to sulfometuron methyl.

### EXAMPLE 8

### Expression and Characterization of NA Protein Produced in Schizochytrium

*Schizochytrium* sp. ATCC 20888 was used as a host cell for transformation with the vectors pCL0140 and pCL0149 with a Biolistic^{™} particle bombarder (BioRad, Hercules, CA), as described in Example 2. The transformants were selected for growth on solid media containing the appropriate antibiotic. gDNA from primary transformants was extracted and purified and used as a template for PCR to check for the presence of the transgene, as described earlier (Example 2).

Cryostocks of transgenic *Schizochytrium* (transformed with pCL0140 and pCL0149) were grown in M50-20 to confluence and then propagated in 50 mL baffled shake flasks as described in Example 2.

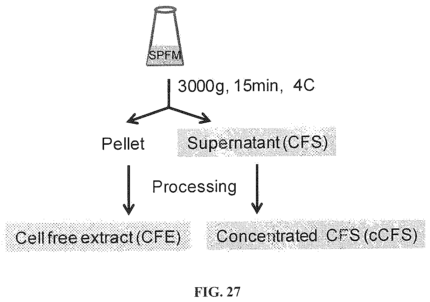

*Schizochytrium* cultures were transferred to 50 mL conical tubes and centrifugated at 3000 × g for 15 min. *See* **FIG. 27****.** The supernatant resulting from this centrifugation, was termed the "cell-free supernatant" (CFS). The CFS fraction was concentrated 50-100 fold using Centriprep^{™} gravity concentrators (Millipore, Billerica, MA) and termed the "concentrated cell-free supernatant" (cCFS). The cell pellet resulting from the centrifugation was washed in water and frozen in liquid nitrogen before being resuspended in twice the pellet weight of lysis buffer (consisting of 50 mM sodium phosphate (pH 7.4), 1 mM EDTA, 5% glycerol, and 1 mM fresh phenylmethylsulphonylfluoride) and twice the pellet weight of 0.5 mm glass beads (Sigma, St. Louis, MO)). The cell pellet mixture was then lysed by vortexing at 4°C in a multi-tube vortexer (VWR, Westchester, PA) at maximum speed for 3 hours. The resulting cell lysate was then centrifuged at 5500 × g for 10 minutes at 4°C. The resulting supernatant was retained and re-centrifuged at 5500 × g for 10 minutes at 4°C. The resulting supernatant is defined herein as "cell-free extract" (CFE). Protein concentration was determined in cCFS and CFE by a standard Bradford assay (Bio-Rad, Hercules, CA). These fractions were used for neuramidase activity assays as well as immunoblot analysis,

A functional influenza NA protein displays neuraminidase activity that can be detected by a standard fluorometric NA activity assay based on the hydrolysis of a sodium (4-Methylumbelliferyl)-α-D-*N*-Acetylneuraminate (4-MUNANA) substrate (Sigma-Aldrich, St. Louis, MO) by sialidases to give free 4-methylumbelliferone which has a fluorescence emission at 450 nm following an excitation at 365 nm. Briefly, the CFS, cCFS or CFE of transgenic *Schizochytrium* strains were assayed following the procedure described by Potier et al., Anal. Biochem. 94: 287-296 (1979), using 25 µL of CFS and 75 µL of 40 µM 4-MUNANA or 75 µL ddH2O for controls. Reactions were incubated for 30 minutes at 37°C and fluorescence was measured with a FLUOstar Omega multimode microplate reader (BMG LABTECH, Offenburg, Germany).

Typical activities observed in concentrated cell-free supernatants (cCFSs) and cell-free extracts (CFEs) from 9 transgenic strains of *Schizochytrium* transformed with CL0140 are presented in **FIG. 28****.** The wild-type strain of *Schizochytrium* sp. ATCC 20888 ("-") and a PCR-negative strain of *Schizochytrium* transformed with pCL0140 ("27"), grown and prepared in the same manner as the transgenic strains, were used as negative controls. The majority of the activity was found in the concentrated cell-free supernatant, indicating the successful expression and secretion of a functional influenza neuraminidase to the outer milieu by *Schizochytrium.*

### Peptide Sequence Analysis

Transgenic *Schizochytrium* strain CL0140-26 was used for partial purification of the influenza NA protein to confirm its successful expression and secretion by peptide sequence analysis. The purification procedure was adapted from Tarigan *et al., JITV 14*(1): 75-82 (2008), and followed by measuring the NA activity (**FIG. 29A**), as described above. Briefly, the cell-free supernatant of the transgenic strain CL0140-26 was further centrifugated at 100,000 × g for 1 hour at 4°C. The resulting supernatant was concentrated 100 fold (fraction "cCFS" in **FIG. 29A**) using Centriprep^{™} gravity concentrators (Millipore, Billerica, MA) and diluted back to the original volume (fraction "D" in **FIG. 29A**) with 0.1M sodium bicarbonate buffer (pH 9.1) containing 0.1% Triton X-100. This diluted sample was used for purification by affinity chromatography. N-(*p-*aminophenyl) oxamic acid agarose (Sigma-Aldrich, St. Louis, MO) was packed into a PD-10 column (BioRad, Hercules, CA), activated by washing with 6 column volumes (CV) of 0.1 M sodium bicarbonate buffer (pH 9.1) containing 0.1% Triton X-100 followed by 5 CV of 0.05 M sodium acetate buffer pH 5.5 containing 0.1% Triton X-100. The diluted sample (fraction "D") was loaded into the column; unbound materials were removed by washing the column with 10 CV of 0.15 M sodium acetate buffer containing 0.1% Triton X-100 (fraction "W" in **FIG. 29A**). Bound NA was eluted from the column with 5CV of 0.1 M sodium bicarbonate buffer containing 0.1% Triton X-100 and 2 mM CaCl2 (fraction "E" in **FIG. 29A**). The NA-rich solution of fraction E was concentrated to about 10% original volume using a 10-kDa-molecular-cut-off-spin concentrator to produce fraction cE.

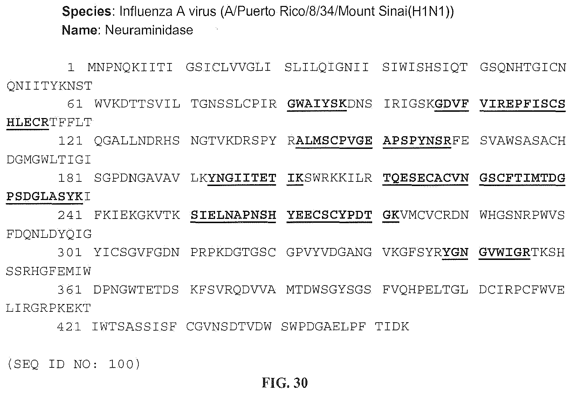

The proteins from each fraction were separated by sodium dodecyl sulfate-polyacrylamide gel electrophoresis (SDS-PAGE) on a NuPAGE^{®} Novex^{®} 12% bis-tris gel (Invitrogen, Carlsbad, CA) under reducing conditions with MOPS SDS running buffer. The proteins were then stained with Coomassie blue (SimplyBlue Safe Stain, Invitrogen, Carlsbad, CA). The proteins bands visible in lane "cE" (**FIG. 29B**) were excised from the Coomassie blue-stained gel and peptide sequence analysis was performed as described in Example 2. The protein band containing NA protein (indicated by the arrow in lane "cE") was identified by a total of 9 peptides (113 amino acids) covering 25% of the protein sequence. The specific peptides that were sequenced are highlighted in bold font in **FIG. 30****.**

### Immunoblot Analysis

The expression of the recombinant NA protein from transgenic *Schizochytrium* CL0149 (clones 10, 11, 12) was tested by immunoblot analysis following standard immunoblotting procedure **(****FIG. 31B****)**. The proteins from the cell-free supernatant (CFS) were separated by sodium dodecyl sulfate-polyacrylamide gel electrophoresis (SDS-PAGE) on a NuPAGE^{®} Novex^{®} 12% bis-tris gel (Invitrogen, Carlsbad, CA) under reducing conditions with MOPS SDS running buffer. The proteins were then stained with Coomassie blue (SimplyBlue Safe Stain, Invitrogen, Carlsbad, CA) or transferred onto polyvinylidene fluoride membrane and probed for the presence of NA protein with anti-V5-AP conjugated mouse monoclonal antibody (1:1000 dilution, #962-25, Invitrogen, Carlsbad, CA). The membrane was then treated with 5-bromo-4-chloro-3-indoyl-phosphate/nitroblue tetrazolium solution (BCIP/NBT) according to the manufacturer's instructions (KPL, Gaithersburg, MD). The recombinant NA protein was detected in the cell-free supernatant of clone 11 (**FIG. 31B**). The negative control ("-") was the wild-type strain of *Schizochytrium* sp. ATCC 20888. The positive control ("+") was the Positope^{™} antibody control protein (#R900-50, Invitrogen, Carlsbad, CA). The corresponding neuraminidase activity is presented in **FIG. 31A****.**

### EXAMPLE 9

### Simultaneous expression of Influenza HA and NA in Schizochytrium

*Schizochytrium* sp. ATCC 20888 was used as a host cell for simultaneous transformation with the vectors pCL0140 (**FIG. 25A**) and pCL0143 (**FIG. 2**) with a Biolistic^{™} particle bombarder (BioRad, Hercules, CA), as described in Example 2.

Cryostocks of transgenic *Schizochytrium* (transformed with pCL0140 and pCL0143) were cultivated and processed as described in Example 2. The hemagglutination and neuraminidase activities were measured as described in Examples 2 and 7, respectively, and are shown in FIG. 32. Transgenic *Schizochytrium* transformed with pCL0140 and pCL0143 demonstrated activities associated with HA and NA.

### EXAMPLE 10

### Expression and Characterization of Extracellular Bodies Comprising Parainfluenza F Protein Produced in Schizochytrium

*Schizochytrium* sp. ATCC 20888 is used as a host cell for transformation with a vector comprising a sequence that encodes the F protein of human parainfluenza 3 virus strain NIH 47885, (GenBank Accession No. P06828). A representative sequence for the F protein is provided as SEQ ID NO: 102. Some cells are transformed with a vector comprising a sequence encoding the native signal peptide sequence associated with the F protein. Other cells are transformed with a vector comprising a sequence encoding a different signal peptide sequence (such as, for example, a *Schizochytrium* signal anchor sequence) that is fused to the sequence encoding the F protein, such that the F protein is expressed with a heterologous signal peptide sequence. Other cells are transformed with a vector comprising a sequence encoding a different membrane domain (such as, for example, a HA membrane domain) that is fused to the sequence encoding the F protein, such that the F protein is expressed with a heterologous membrane domain. The F protein comprises a single-pass transmembrane domain near the C-terminus. The F protein can be split into two peptides at the Furin cleavage site (amino acid 109). The first portion of the protein designated F2 contains the N-terminal portion of the complete F protein. The F2 region can be fused individually to sequences encoding heterologous signal peptides. The remainder of the viral F protein containing the C-terminal portion of the F protein is designated F1. The F1 region can be fused individually to sequences encoding heterologous signal peptides. Vectors containing the F1 and F2 portions of the viral F protein can be expressed individually or in combination. A vector expressing the complete F protein can be co-expressed with the furin enzyme that will cleave the protein at the furin cleavage site. Alternatively, the sequence encoding the furin cleavage site of the F protein can be replaced with a sequence encoding an alternate protease cleavage site that is recognized and cleaved by a different protease. The F protein containing an alternate protease cleavage site can be co-expressed with a corresponding protease that recognizes and cleaves the alternate protease cleavage site.

Transformation is performed, and cryostocks are grown and propagated according to any of the methods described herein. *Schizochytrium* cultures are transferred to 50 mL conical tubes and centrifugated at 3000 × g or 4500 × g for 15 min to yield a low-speed supernatant. The low-speed supernatant is further ultracentrifugated at 100,000 × g for 1 h. *See* **FIG. 3****.** The resulting pellet of the insoluble fraction containing the F protein is resuspended in phosphate buffer saline (PBS) and used for peptide sequence analysis as well as glycosylation analysis as described in Example 2.

The expression of the F protein from transgenic *Schizochytrium* is verified by immunoblot analysis following standard immunoblotting procedure as described in Example 2, using anti-F antiserum and a secondary antibody at appropriate dilutions. The recombinant F protein is detected in the low-speed supernatant and the insoluble fraction. Additionally, the recombinant F protein is detected in cell-free extracts from transgenic *Schizochytrium* expressing the F protein.

The activity of the F protein produced in *Schizochytrium* is evaluated by a F activity assay. A functional F protein displays an F activity that is readily detected by a standard F activity assay.

Electron microscopy, using negative-stained material produced according to Example 4, is performed to confirm the presence of extracellular bodies. Immunogold labeling is performed to confirm the association of protein with extracellular membrane bodies.

### EXAMPLE 11

### Expression and Characterization of Extracellular Bodies Comprising G Vesicular Stomatitus Virus G Protein Produced in Schizochytrium

*Schizochytrium* sp. ATCC 20888 is used as a host cell for transformation with a vector comprising a sequence that encodes the Vesicular Stomatitis virus G (VSV-G) protein. A representative sequence for the VSV-G protein is provided as SEQ ID NO: 103 (from GenBank Accession No. M35214). Some cells are transformed with a vector comprising a sequence encoding the native signal peptide sequence associated with the VSV-G protein. Other cells are transformed with a vector comprising a sequence encoding a different signal peptide sequence (such as, for example, a *Schizochytrium* signal anchor sequence) that is fused to the sequence encoding the VSV-G protein, such that the VSV-G protein is expressed with a heterologous signal peptide sequence. Other cells are transformed with a vector comprising a sequence encoding a different membrane domain (such as, for example, a HA membrane domain) that is fused to the sequence encoding the VSV-G protein, such that the VSV-G protein is expressed with a heterologous membrane domain. Transformation is performed, and cryostocks are grown and propogated according to any of the methods described herein. *Schizochytrium* cultures are transferred to 50 mL conical tubes and centrifugated at 3000 × g or 4500 × g for 15 min to yield a low-speed supernatant. The low-speed supernatant is further ultracentrifugated at 100,000 × g for 1 h. *See* **FIG. 3****.** The resulting pellet of the insoluble fraction containing the VSV-G protein is resuspended in phosphate buffer saline (PBS) and used for peptide sequence analysis as well as glycosylation analysis as described in Example 2.

The expression of the VSV-G protein from transgenic *Schizochytrium* is verified by immunoblot analysis following standard immunoblotting procedure as described in Example 2, using anti- VSV-G antiserum and a secondary antibody at appropriate dilutions. The recombinant VSV-G protein is detected in the low-speed supernatant and the insoluble fraction. Additionally, the recombinant VSV-G protein is detected in cell-free extracts from transgenic *Schizochytrium* exprsesing the VSV-G protein.

The activity of the VSV-G protein produced in *Schizochytrium* is evaluated by a VSV-G activity assay. A functional VSV-G protein displays an VSV-G activity that is readily detected by a standard VSV-G activity assay.

Electron microscopy, using negative-stained material produced according to Example 4, is performed to confirm the presence of extracellular bodies. Immunogold labeling is performed to confirm the association of protein with extracellular membrane bodies.

### EXAMPLE 12

### Expression and Characterization of Extracellular Bodies Comprising eGFP Fusion Proteins Produced in Schizochytrium

Transformation of *Schizochytrium* sp. ATCC 20888 with vectors comprising a polynucleotide sequence encoding eGFP and expression of eGFP in transformed *Schizochytrium* has been described. *See* U.S. Publ. No. 2010/0233760 and WO 2010/107709.

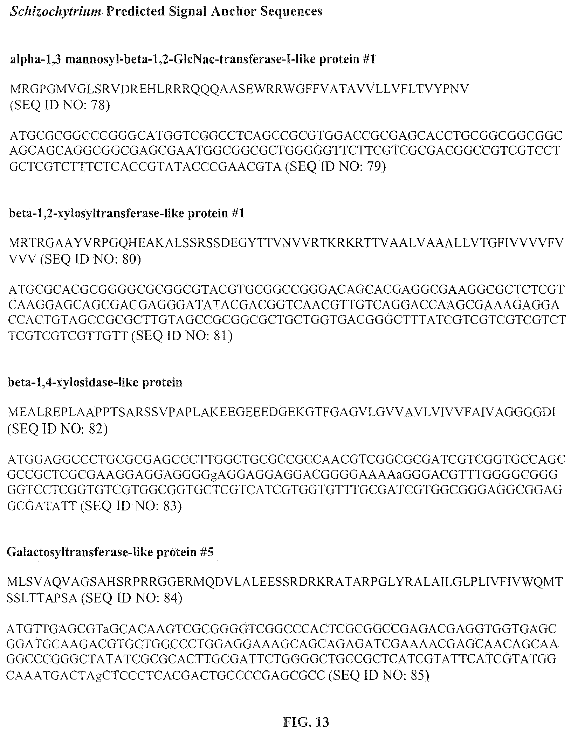

In a future experiment, *Schizochytrium* sp. ATCC 20888 is used as a host cell for transformation with a vector comprising a sequence that encodes a fusion protein between eGFP and a membrane domain, such as, for example, a membrane domain from *Schizochytrium* or a viral membrane domain such as the HA membrane domain. Representative *Schizochytrium* membrane domains are provided in **FIG. 13** and **FIG. 14****.** Transformation is performed, and cryostocks are grown and propogated according to any of the methods described herein. *Schizochytrium* cultures are transferred to 50 mL conical tubes and centrifugated at 3000 × g or 4500 × g for 15 min to yield a low-speed supernatant. The low-speed supernatant is further ultracentrifugated at 100,000 × g for 1 h. *See* **FIG. 3****.** The resulting pellet of the insoluble fraction containing the eGFP fusion protein from transgenic *Schizochytrium* is resuspended in phosphate buffer saline (PBS) and used for peptide sequence analysis as well as glycosylation analysis as described in Example 2.

The expression of the eGFP fusion protein from transgenic *Schizochytrium* is verified by immunoblot analysis following standard immunoblotting procedure as described in Example 2, using anti-eGFP fusion protein antiserum and a secondary antibody at appropriate dilutions. The recombinant eGFP fusion protein is detected in the low-speed supernatant and the insoluble fraction. Additionally, the recombinant eGFP fusion protein is detected in cell-free extracts from transgenic *Schizochytrium* expressing the eGFP fusion protein.

The activity of the eGFP fusion protein produced in *Schizochytrium* is evaluated by a eGFP fusion protein activity assay. A functional eGFP fusion protein displays an eGFP fusion protein activity that is readily detected by a standard eGFP fusion protein activity assay.

Electron microscopy, using negative-stained material produced according to Example 4, is performed to confirm the presence of extracellular bodies. Immunogold labeling is performed to confirm the association of protein with extracellular membrane bodies.

### EXAMPLE 13

### Detection of Heterologous Polypeptides Produced in Thraustochytrid Cultures

A culture of a thraustochytrid host cell is prepared comprising at least one heterologous polypeptide in a fermentor under appropriate fermentation conditions. The fermentor is batched with a media containing, for example, carbon (glucose), nitrogen, phosphorus, salts, trace metals, and vitamins. The fermentor is inoculated with a typical seed culture, then cultivated for 72 - 120 hours, and fed a carbon (e.g., glucose) feed. The carbon feed is fed and consumed throughout the fermentation. After 72 - 120 hours, the fermentor is harvested and the broth is centrifuged to separate the biomass from the supernatant.

The protein content is determined for the biomass and the cell-free supernatant by standard assays such as Bradford or BCA. Proteins are further analyzed by standard SDS-PAGE and Western blotting to determine the expression of the heterologous polypeptide(s) in the respective biomass and cell-free supernatant fractions. The heterologous polypeptide(s) comprising membrane domains are shown to be associated with microalgal extracellular bodies by routine staining procedures (e.g., negative staining and immunogold labeling) and subsequent electron microscope observations.

### EXAMPLE 14

### Preparation of Virus-Like Particles from Microalgal Cultures

One or more viral envelope polypeptides are heterologously expressed in a microalgal host cell under conditions described above, such that the viral polypeptides are localized to microalgal extracellular bodies produced under the culture conditions. When overexpressed using appropriate culture conditions and regulatory control elements, the viral envelope polypeptides in the microalgal extracellular bodies spontaneously self-assemble into particles that are morphologically similar to infectious virus.

Similarly, one or more viral envelope polypeptides and one or more viral matrix polypeptides are heterologously expressed in a microalgal host cell under conditions described above, such that the viral polypeptides are localized to microalgal extracellular bodies produced under the culture conditions. When overexpressed using appropriate culture conditions and regulatory control elements, the viral polypeptides in the microalgal extracellular bodies spontaneously self-assemble into particles that are morphologically similar to infectious virus.

All of the various aspects, embodiments, and options described herein can be combined in any and all variations.

## Claims

1. A method for production of a viral neuraminidase (NA) protein, comprising:
culturing a recombinant microalgal cell in a medium such that the NA protein is secreted into the medium, wherein the recombinant microalgal cell comprises a nucleic acid molecule comprising a polynucleotide sequence that encodes the viral NA protein, wherein the viral NA protein comprises a membrane domain; and
recovering the secreted recombinant viral NA protein from the medium.

2. The method of claim 1, wherein the viral NA protein is a full-length viral NA protein.

3. The method of claim 1 or claim 2, wherein the viral NA protein is an influenza NA protein.

4. The method of claim 1, wherein the viral NA protein comprises an amino acid sequence that is at least 90% identical to the amino acid sequence encoded by SEQ ID NO: 100.

5. The method of claim 1, wherein the viral NA protein has the amino acid sequence encoded by SEQ ID NO: 100.

6. The method of any one of claims 1 to 5, wherein the microalgal cell is a member of the phylum Labyrinthulomycota.

7. The method of any one of claims 1 to 6, wherein the microalgal cell is a member of the order Thraustochytriales.

8. The method of any one of claims 1 to 7, wherein the microalgal cell is a Schizochytrium or a Thraustochytrium cell.

9. The method of any one of claims 1-8, wherein the viral NA protein is a glycoprotein.

10. The method of claim 9, wherein the glycoprotein comprises an N-linked glycosylation pattern.

11. The method of claim 9, wherein the glycoprotein comprises high-mannose oligosaccharides.

12. The method of claim 9, wherein the glycoprotein is substantially free of sialic acid.

## Patentansprüche

1. Verfahren zur Erzeugung eines viralen Neuraminidase(NA)-Proteins, umfassend:
Kultivieren einer rekombinanten Mikroalgenzelle in einem Medium, so dass das NA-Protein in das Medium sezerniert wird, wobei die rekombinante Mikroalgenzelle ein Nukleinsäuremolekül umfasst, das eine Polynukleotidsequenz umfasst, die das virale NA-Protein codiert, wobei das virale NA-Protein eine Membrandomäne umfasst; und
Gewinnen des sezernierten rekombinanten viralen NA-Proteins aus dem Medium.

2. Verfahren nach Anspruch 1, wobei es sich bei dem viralen NA-Protein um ein virales NA-Protein in voller Länge handelt.

3. Verfahren nach Anspruch 1 oder Anspruch 2, wobei es sich bei dem viralen NA-Protein um ein Influenza-NA-Protein handelt.

4. Verfahren nach Anspruch 1, wobei das virale NA-Protein eine Aminosäuresequenz umfasst, die mit der durch SEQ ID NO: 100 codierten Aminosäuresequenz zu wenigstens 90% identisch ist.

5. Verfahren nach Anspruch 1, wobei das virale NA-Protein die durch SEQ ID NO: 100 codierte Aminosäuresequenz aufweist.

6. Verfahren nach einem der Ansprüche 1 bis 5, wobei es sich bei der Mikroalgenzelle um ein Mitglied des Phylums Labyrinthulomycota handelt.

7. Verfahren nach einem der Ansprüche 1 bis 6, wobei es sich bei der Mikroalgenzelle um ein Mitglied der Ordnung Thraustochytriales handelt.

8. Verfahren nach einem der Ansprüche 1 bis 7, wobei es sich bei der Mikroalgenzelle um eine Schizochytrium- oder eine Thraustochytrium-Zelle handelt.

9. Verfahren nach einem der Ansprüche 1-8, wobei es sich bei dem viralen NA-Protein um ein Glykoprotein handelt.

10. Verfahren nach Anspruch 9, wobei das Glykoprotein ein N-verknüpftes Glykosylierungsmuster umfasst.

11. Verfahren nach Anspruch 9, wobei das Glykoprotein mannosereiche Oligosaccharide umfasst.

12. Verfahren nach Anspruch 9, wobei das Glykoprotein weitgehend frei von Sialinsäure ist.

## Revendications

1. Procédé pour la production d'une protéine de neuraminidase (NA) virale, comprenant:
la culture d'une cellule de microalgue recombinante dans un milieu de manière que la protéine NA est sécrétée dans le milieu, la cellule de microalgue recombinante comprenant une molécule d'acide nucléique comprenant une séquence de polynucléotides qui code pour la protéine NA virale, la protéine NA virale comprenant un domaine membranaire; et
la récupération de la protéine NA virale recombinante sécrétée du milieu.

2. Procédé selon la revendication 1, la protéine NA virale étant une protéine NA virale de pleine longueur.

3. Procédé selon la revendication 1 ou la revendication 2, la protéine NA virale étant une protéine NA de la grippe.

4. Procédé selon la revendication 1, la protéine NA virale comprenant une séquence d'acides aminés qui est au moins 90% identique à la séquence d'acides aminés codée par la SEQ ID NO: 100.

5. Procédé selon la revendication 1, la protéine NA virale ayant la séquence d'acides aminés codée par la SEQ ID NO: 100.

6. Procédé selon l'une quelconque des revendications 1 à 5, la cellule de microalgue étant un membre du phylum Labyrinthulomycota.

7. Procédé selon l'une quelconque des revendications 1 à 6, la cellule de microalgue étant un membre de l'ordre des Thraustochytriales.

8. Procédé selon l'une quelconque des revendications 1 à 7, la cellule de microalgue étant une cellule de Schizochytrium ou une cellule de Thraustochytrium.

9. Procédé selon l'une quelconque des revendications 1 à 8, la protéine NA virale étant une glycoprotéine.

10. Procédé selon la revendication 9, la glycoprotéine comprenant un motif de glycosylation N-lié.

11. Procédé selon la revendication 9, la glycoprotéine comprenant des oligosaccharides à haute teneur en mannose.

12. Procédé selon la revendication 9, la glycoprotéine étant sensiblement exempte d'acide sialique.
